# EUROPÄISCHE PATENTSCHRIFT

(11) **EP 1 144 590 B1**
(45) Veröffentlichungstag und Bekanntmachung des Hinweises auf die Patenterteilung: **31.12.2014**
(21) Anmeldenummer: 00904978.4
(22) Anmeldetag: 28.01.2000
(51) Int. Cl.: C12N 1/21, C12N 15/62, C12N 15/31, C07K 14/32, C12N 15/54, C12N 9/10, C12N 15/70, A61K 39/00, A61K 35/74, C12R 1/19

(54) **KOMPARTIMENTIERUNG VON REKOMBINANTEN POLYPEPTIDEN IN WIRTSZELLEN**
COMPARTMENTALIZATION OF RECOMBINANT POLYPEPTIDES IN HOST CELLS
COMPARTIMENTAGE DE POLYPEPTIDES RECOMBINANTS DANS DES CELLULES HOTES

(30) Priorität: 28.01.1999 DE 19903345
(43) Veröffentlichungstag der Anmeldung: 17.10.2001
(73) Patentinhaber: Prof. Dr. Lubitz, Werner, 3420 Klosterneuburg/Kritzendorf (AT)
(72) Erfinder: Prof. Dr. Lubitz, Werner, 3420 Klosterneuburg/Kritzendorf (AT)
(74) Vertreter: Dey, Michael
(86) Internationale Anmeldenummer: PCT/EP2000/000686
(87) Internationale Veröffentlichungsnummer: WO 2000/044878

(56) Entgegenhaltungen:
- WO-A-93/10246
- WO-A-97/28263
- WO-A1-91/13155
- WO-A1-99/06567
- DE-A- 4 417 169
- VALLS M. ET AL.: "Bioaccumulation of heavy metals with protein fusions of metallothionein to bacterial OMPs." BIOCHIMIE, Bd. 80, Nr. 10, Oktober 1998 (1998-10), Seiten 855-861, XP000911106 ISSN: 0300-9084
- SLEYTR U. B. ET AL.: "BACTERIAL AND ARCHAEAL S-LAYER PROTEINS: STRUCTURE-FUNCTION RELATIONSHIPS AND THEIR BIOTECHNOLOGICAL APPLICATIONS" TRENDS IN BIOTECHNOLOGY, Bd. 15, Nr. 1. (156), 1. Januar 1997 (1997-01-01), Seiten 20-26, XP002032144 ISSN: 0167-7799
- SZOSTAK M. P. ET AL.: "Bacterial ghosts as multifunctional vaccine particles." BEHRING INSTITUTE MITTEILUNGEN, Nr. 98, 1997, Seiten 191-196, XP000907215 ISSN: 0301-0457
- LUBITZ W. ET AL.: "Extended recombinant bacterial ghost system." JOURNAL OF BIOTECHNOLOGY, Bd. 73, Nr. 2-3, 20. August 1999 (1999-08-20), Seiten 261-273, XP002138570 ISSN: 0168-1656
- DATABASE MEDLINE [Online] US NATIONAL LIBRARY OF MEDICINE (NLM), BETHESDA, MD, US 1997 JOBLING M G ET AL: 'Construction and characterization of versatile cloning vectors for efficient delivery of native foreign proteins to the periplasm of Escherichia coli.' Database accession no. NLM9435018
- DATABASE MEDLINE [Online] US NATIONAL LIBRARY OF MEDICINE (NLM), BETHESDA, MD, US Juli 2000 FEILMEIER B J ET AL: 'Green fluorescent protein functions as a reporter for protein localization in Escherichia coli.' Database accession no. NLM10869087
- DATABASE MEDLINE [Online] US NATIONAL LIBRARY OF MEDICINE (NLM), BETHESDA, MD, US Februar 1995 SNYDER W B ET AL: 'Beta-galactosidase is inactivated by intermolecular disulfide bonds and is toxic when secreted to the periplasm of Escherichia coli.' Database accession no. NLM7860606

## Beschreibung

Die vorliegende Erfindung betrifft Gram-Negative Bakterien wirtszellen umfassend mindestens zwei verschiedene funktionelle heterologe rekombinante Polypeptide, von denen mindestens eines in trägergebundener Form als fusion mit einem S-Layer-Struktur Polypeptid vorliegt, wobei die verschiedenen funktionellen heterologen rekombinanten Polypeptide in jeweils unterschiedlichen Zellkompartimenten, z.B. Cytosol, cytoplasmatische Membran, Periplasma und Außenmembran lokalisiert sind, sowie Verfahren zu ihrer Herstellung. Die erfindungsgemäßen Zellen eignen sich insbesondere als Bioreaktoren zur Durchführung enzymatischer Reaktionskaskaden, bei denen eine Kompartimentierung einzelnder Enzyme von Vorteil oder erforderlich ist.

Die Verwendung lebender Zellen als "Enzymreaktoren" zur Herstellung von biologischen Substanzen, z.B. Polyhydroxyfettsäuren, ist für die biotechnologische Industrie von hoher Bedeutung. Hierzu ist es bekannt, Fremdgene in eine Wirtszelle einzuführen und zu exprimieren, um auf diese Weise eine Wirtszelle mit rekombinanten Enzymen zu erhalten, die in der Lage ist, ein gewünschtes Produkt zu synthetisieren. Ein Nachteil bekannter Verfahren bestand jedoch darin, daß die in den Wirtszellen durch Expression der Fremdgene erzeugten Enzyme nicht stabil, zu wenig aktiv oder in zu geringer Menge vorhanden waren. Besondere Schwierigkeiten traten auch bei der Durchführung mehrstufiger Reaktionen, in denen Substrate oder Produkte der einen Stufe negativ auf andere Stufen wirken können.

Aufgabe der vorliegenden Erfindung war es, die Probleme des Standes der Technik mindestens teilweise zu beseitigen und Wirtszellen bereitzustellen, die in der Lage sind, funktionelle rekombinante Polypeptide stabil zu präsentieren und insbesondere mehrstufige Enzymreaktionen durchzuführen.

Die Aufgabe wird gelöst durch Bereitstellung einer gram-negativen bakterien wirtszelle umfassend mindestens zwei verschiedene funktionelle heterologe rekombinante Polypeptide, von denen mindestens eines in trägergebundener Form als fusion mit einem S-Layer-Struktur Polypeptid vorliegt, wobei die verschiedenen funktionellen heterologen rekombinanten Polypeptide in jeweils unterschiedlichen Kompartimenten davon lokalisiert sind.

Überraschenderweise wurde festgestellt, daß die trägergebundene rekombinante Expression von heterologen Polypeptiden in unterschiedlichen Kompartimenten von gram-negativen Bakterienzellen, zu einer stabilen Präsentation der heterologen, Polypeptide in funktioneller (d.h. immunologisch oder/und biologisch, z.B. enzymatisch aktiver) Form führt. Wenn die Wirtszelle eine gram-negative Bakterienzelle ist, können die Zellkompartimente vorzugsweise ausgewählt werden aus dem Cytosol, der cytoplasmatischen Membran (Außen- und Innenseite), dem periplasmatischen Raum und der Außenmembran (Außen- und Innenseite).

Die in der Wirtszelle vorliegenden funktionellen rekombinanten Proteine sind vorzugsweise kooperativ, d.h. sie erfüllen eine gemeinsame immunologische oder/und biologische Funktion, z.B. als Enzyme in einer mehrstufigen Reaktionskaskade.

Mindestens eines der funktionellen rekombinanten Polypeptide, vorzugsweise mehrere, liegen in einer trägergebundenen Form vor, beispielsweise in Form von Fusionspolypeptiden und enthalten mindestens eine funktionelle Domäne und mindestens eine Trägerdomäne. Bevorzugte Formen trägergebundener Polypeptide sind S-Layer-Strukturen (Fusionspolypeptide mit S-Layer-Träger-Domänen), membrangebundene Polypeptide (Fusionspolypeptide mit Membran-integrierenden Trägerdomänen) oder/und Bestandteile rekombinanter Phagenstrukturen. Falls ein Export der funktionellen Polypeptide vom Cytosol in andere Kompartimente der Zelle gewünscht ist, erfolgt die Expression zusammen mit geeigneten Targetingdomänen, die einen Export in das jeweils gewünschte Zellkompartiment ermöglichen. Beispiele für Targetingdomänen sind Signalpeptid- oder/und Helfersequenzen, die den Durchtritt durch Membranen ermöglichen.

Erfindungsgemäß ist mindestens eines der trägergebundenen Polypeptide als rekombinante S-Layer Struktur vorhanden. S-Layer sind kristalline bakterielle Zelloberflächenproteine, die aus identischen selbst-assemblierenden Einheiten aufgebaut sind. Genetische Daten und Sequenzinformationen für verschiedene S-Layer-Gene aus Mikroorganismen sind beispielsweise bei Peyret et al. (Mol. Microbiol. 9 (1993), 97-109) angebeben.

Bevorzugte S-Layer-Gene sind die Gene sbsA und sbsB von B.stearothermophilus PV72. Die Sequenz dieser Gene ist beispielsweise in der internationalen Patentanmeldung WO 9 728 263 angegeben. Dort wird auch die Herstellung eines rekombinanten S-Layer-Fusionsproteins im Cytoplasma gram-negativer Wirtszellen offenbart. Die internationale Patentameldung WO 9 906 567 und das Dokument S20STAK M.P. et al (1997), Behring Inst. Mitt. No. 98, 191-196 beschreiben wiederum die Herstellung eines rekombinanten S-Layer-Proteihs in verschiedenen Kompartimenten von gram-negativen Bakterienzellen. Bezüglich der Konstruktion von rekombinanten S-Layer-Genen und der Herstellung geeigneter Expressionskonstrukte wird ausdrücklich auf diese zwei genannten internationalen Anmeldungen verwiesen. Es findet sich dort jedoch kein Hinweis auf die Coexpression zweier verschiedener funktioneller rekombinanter Polypeptide.

Überraschenderweise wurde festgestellt, daß eine gleichzeitige oder/und sequentielle Coexpression von mehreren rekombinanten S-Layer-Proteinen gegebenenfalls in Kombination mit weiteren heterologen Proteinen, z.B. in verschiedenen Kompartimenten von Wirtszellen, insbesondere von gram-negativen Bakterienzellen möglich ist.

Die Nukleotidsequenz des für das reife SbsA-Protein kodierenden Gens ist in SEQ ID No. 1 von Position 91-3684 angegeben. Die zugehörige Aminosäuresequenz ist in SEQ ID No. 2 dargestellt. Die Nukleotidsequenz des für das reife SbsB-Protein kodierenden Gens ist in SEQ ID No. 3 von Position 94-2763 angegeben. Die zugehörige Aminosäuresequenz ist in SEQ ID No. 4 dargestellt.

In einer ersten bevorzugten Ausführungsform (sbsA) wird die Nukleinsäure, die für die Trägerdomäne eines funktionellen Polypeptids kodiert, ausgewählt aus
(i) einer Nukleinsäure, welche die von Position 91 bis 3684 in SEQ ID No. 1 gezeigten Nukleotidsequenz umfaßt,
(ii) einer Nukleinsäure, welche eine der Nukleinsäure aus (i) im Rahmen der Degeneration des genetischen Codes entsprechende Nukleotidsequenz umfaßt, und
(iii) einer Nukleinsäure, welche eine mit den Nukleinsäuren aus (i) oder/und (ii) unter stringenten Bedingungen hybridisierende Nukleotidsequenz umfaßt.

In einer zweiten bevorzugten Ausführungsform (sbsB) wird die Nukleinsäure, die für die Trägerdomäne eines funktionellen Polypeptids kodiert, ausgewählt aus
(i) einer Nukleinsäure, welche die von Position 94 bis 2763 in SEQ ID No. 3 gezeigte Nukleotidsequenz umfaßt,
(ii) einer Nukleinsäure, welche eine der Nukleinsäure aus (i) im Rahmen der Degeneration des genetischen Codes entsprechenden Nukleotidsequenz umfaßt und
(iii) einer Nukleinsäure, welche eine mit den Nukleinsäuren aus (i) oder/und (ii) unter stringenten Bedingungen hybridisierende Nukleotidsequenz umfaßt.

Unter dem Begriff "stringente Hybridisierung" im Sinne der vorliegenden Erfindung versteht man, daß eine Hybridisierung auch nach Waschen bei 55°C, vorzugsweise 60°C, in einem wäßrigen Niedrigsalz-Puffer (z.B. 0,2 x SSC) noch auftritt (s. auch Sambrook et al. (1989), Molecular Cloning. A Laboratory Manual).

Als Insertionsstellen für Peptid- oder Polypeptid-kodierende Sequenzen in das sbsA-Gen bevorzugt sind Bereiche zwischen den Positionen 200-3600 der in SEQ ID NO.1 gezeigten Nukleotidsequenz. Besonders bevorzugte Insertionsstellen sind die Nrul-Schnittstelle an Position 585, die Pvull-Schnittstelle an Position 881, die SnaB-I-Schnittstelle an Position 920, die Pvull-Schnittstelle an Position 2507 und die Pvull-Schnittstelle an Position 2652 (WO 9 728 263). Weitere bevorzugte Insertionsstellen sind die Positionen 562, 1087, 1813, 1947, 2295, 2652, 3046, 3484 und 3594. Die jeweils angegebenen Positionen beziehen sich auf das erste Nukleotid der Insertion.

Als Insertionsstellen in das sbsB-Gen bevorzugt sind Bereiche zwischen den Positionen 200-2600 der in SEQ ID No. 3 gezeigten Nukleotidsequenz. Besonders bevorzugte Insertionsstellen sind die Positionen 410 (Codon 136), 484 (Codon 161/162) und 1583 (Codon 528/529) (WO 9 728 263). Weitere bevorzugte Insertionsstellen sind die Positionen 598, 1012, 1435, 1808 und 2301, wobei sich die jeweils angegebene Position auf das erste Nukleotid der Insertion bezieht.

Alternativ oder zusätzlich können trägergebundene Polypeptide auch in anderer Form hergestellt werden, z.B. als Bestanteile rekombinanter Phagenstrukturen, z.B. *φ*X174 oder *φ*CH1.

Noch eine weitere Möglichkeit der Herstellung von trägergebundenen Polypeptiden ist die Synthese als Fusionspolypeptide mit Membranintegrationsdomänen, so daß die funktionellen Polypeptide an jeweils gewünschten Orten (Außen- bzw. Innenseite einer jeweils gewählten Membran) lokalisiert sind.

Bei einer Integration in die äußere Membran prokaryontischer gram-negativer Wirtszellen kann als Trägersequenz die C-terminale Domäne der IgA-Protease aus Neisseria oder Haemophilus (Klauser et al., J. Mol. Bio. 234 (1993), 579-593) verwendet werden. Weitere geeignete Trägerdomänensequenzen sind die OmpA- oder LamB-Sequenzen bzw. Teile davon.

Bei einer Integration in die cytoplasmatische Membran gram-negativer prokaryontischer Wirtszellen wird vorzugsweise ein hydrophobe, nicht lytisch wirkende membranintegrierende Proteindomäne, die eine *α*-helikale Struktur besitzt, verwendet. Beispiele von DNA-Sequenzen, die für eine solche membranintegrierende Proteindomäne kodieren, sind im europäischen Patent 0 516 655 beschrieben.

Bei einer Sekretion ins Periplasma gram-negativer prokaryontischer Zellen kann z.B. die malE-Signalpeptidsequenz verwendet werden. Andere Sequenzen, die eine Sekretion ins Periplasma bewirken, sind beispielsweise bei Blondel und Bedouelle (Eur. J. Biochem 193 (1990), 325-330; Adip-Conquy et al. (Protein Eng. 8 (1995), 859-863); Weller et al (Eur. J. Biochem. 236 (1996), 34-39) und Dubreuil et al. (FEMS Immunol. Med. Microbiol. 13 (1996), 317-323) beschrieben.

Für die Expression in der cytoplasmatischen Membran oder in Organellen eukaryontischer Zellen sind als Signalpeptide das N-terminale Transitpeptid von Plastocyanin für den Transport in Chloroplasten (Weisbeek et al., J. Cell. Sci. Suppl. 11 (1989), 199-223), mitrochondriale Signalpeptide für den Transport in Mitochondrien (Skerjanc, Biochem. Cell. Biol. 68 (1990), 9-16), Targetingsequenzen für den Transport in Vakuolen (Vitale und Chrispeels, Bioessays 14 (1992), 151-160), Targetingsequenzen für die Zellmembran, das Cytoplasma und den Golgiapparat (Stanley, Mol. Membr. Biol. 13 (1996), 19-27), Retentionssignale für das endoplasmatische Reticulum (Lencer et al., J. Cell. Biol. 131 (1995), 951-962) und Transfersequenzen für den Golgiapparat oder die Plamsmamembran (Rambourg et al., Anat. Rec. 245 (1996), 447-458) bekannt.

Neben dem für das Signalpeptid kodierenden Abschnitt kann die für das Fremdpolypeptid kodierende DNA-Sequenz einen oder mehrere weitere Abschnitte enthalten, die für weitere Proteindomänen kodieren. Ein solcher Abschnitt kann vorzugsweise zwischen dem für das Signalpeptid kodierenden Abschnitt und dem für das Fremdpolypeptid kodierenden Abschnitt angeordnet sein. Vorzugsweise kodiert dieser Abschnitt für ein sekretorisches Polypeptid aus gram-negativen bakteriellen oder einen Teil davon. Ein bevorzugtes Beispiel für einen solchen Nukleinsäureabschnitt ist das malE Gen, welches das Maltose-Bindeprotein kodiert.

Die Fremdpolypeptide werden vorzugsweise ausgewählt aus DNA-bindende Epitopen, antigenen, allergenen oder immunogenen Epitopen, metallbindenden Epitopen, Streptavidin, Enzymen, Cytokinen oder Antikörper-bindenden Proteinen.

Ein bevorzugtes Beispiel ist Streptavidin, das z.B. nach Integration in die Außenmembran zur Ankopplung von biotinylierten Reagenzien geeignet ist.

Ein weiteres bevorzugtes Beispiel sind antigene, allergene oder immunogene Epitope, z.B. Epitope aus pathogenen Mikroorganismen, wie etwa Bakterien, Pilzen, Parasiten etc. und Viren, oder Epitope aus Pflanzen oder Epitope gegen körpereigene Substanzen, z.B. Cytokine, sowie gegen Toxine, insbesondere Endotoxine. Besonders bevorzugte Beispiele für immunogene Epitope sind Epitope aus Viren, z.B. aus Herpesviren, wie etwa Herpesvirus 1, z.B. Glykoprotein Δ, Herpesvirus 6 oder Pseudorabiesvirus (Lomniczi et al., J. Virol. 49 (1984), 970-979), insbesondere Epitope aus den Genen gB, gC oder/und gD, Epitope aus Maul- und Klauenseuchevirus (FMDV), insbesondere Epitope aus den Genabschnitten, die für VP1, VP2 oder/und VP3 kodieren, Epitope aus Flaviviren oder Epitope aus Filoviren wie etwa Ebola-, Marburg- oder Lassavirus. Die immunogenen Epitope können so ausgewählt werden, daß sie die Erzeugung einer Antikörper-vermittelten Immunreaktion fördern oder/und die Erzeugung einer zellulären Immunreaktion, z.B. durch Stimulation von T-Zellen, fördern. Beispiele für geeignete allergene Epitope sind Birkenpollenallergene, z.B. Bet v I (Ebner et al., J. Immunol. 150 (1993) 1047-1054). Weiterhin besonders bevorzugt sind antigene Epitope, die in der Lage sind, aus Serum oder anderen Körperflüssigkeiten körpereigene oder körperfremde Substanzen wie etwa Cytokine oder Toxine zu binden und herauszufiltrieren. Derartige Epitope können Bestandteile von Cytokin- oder Toxinrezeptoren oder von Antikörpern gegen Cytokine oder Toxine umfassen.

Modifizierte Fremdpolypeptide, z.B. S-Layer-Proteine, die immunogene oder/und antigene Epitope mit Glykosilierungsstellen aufweisen, können in eukaryontischen Wirtszellen hergestellt werden, in denen eine Glykosilierung möglich ist. Dabei können auch die natürlichen S-Layer-Sequenzen glykosiliert werden. Beispiele für potentielle N-Glykosilierungsstellen im S-Layer-Gen sbsA sind die Aminosäurepositionen 26, 285, 343, 384, 387, 388, 418, 421, 483, 653, 675, 902, 924, 1048, 1058, 1118, 1154 und 1161. Im sbsB-Gen kann eine potentielle N-Glykosilierung an den Positionen 155, 184, 213, 302, 303, 400, 463, 606, 755 und 915 stattfinden. Weitere mögliche Modifikationen des sbsA-Gens umfassen Amidierung, Phosphorylierung durch Caseinkinase II, N-Myristoylierung und Phosphorylierung durch Proteinkinase C. Weitere mögliche Modifikationen des sbsB Gens umfassen Phosphorylierung durch cAMP- und cGMP-abhängige Proteinkinase, Phosphorylierung durch Caseinkinase II, N-Myristoylierung, Phosphorylierung durch Proteinkinase C und Anlagerung an einen Fibronectin Rezeptor (über Sequenz RGD).

Ebenfalls bevorzugt als Fremdpolypeptide sind Cytokine, wie etwa Interleukine, Interferone oder Tumornekrosefaktoren. Diese Moleküle können beispielsweise in Kombination mit immunogenen Epitopen zur Herstellung von Vakzinen verwendet werden. Außerdem sind auch Antikörper-bindende Proteine bevorzugt Protein-A oder Protein-G oder für DNA- oder/und metallbindende Epitope, wie etwa Leucin-Zipper, Zinkfinger etc.

Besonders bevorzugt sind die rekombinanten Polypeptide Enzyme, insbesondere Enzyme, die eine mehrstufige enzymatische Reaktion katalysieren. Spezifische Beispiele sind Enzyme zur Synthese von Polyhydroxyalkanoaten, z.B. die Polyhydroxybuttersäure-Synthase (Lubitz und Resch, DE 44 171 69 A1; Slater, S.C., Voige W.H., Dennis, A.E. J. Bacteriol. (1988), 170:4431).

Noch ein weiterer Gegenstand der vorliegenden Erfindung sind rekombinante Bakterienghosts erhältlich aus einer erfindungsgemäßen gram-negativen Wirtszelle mit mindestens zwei verschiedenen funktionellen heterologen rekombinanten Polypeptiden, von denen mindestens eines in Trägergebundener form als fusion mit einem S-Layer-Struktur Polypeptid vorliegt, wobei die verschiedenen funktionellen heterologen rekombinanten Polypeptide in jeweils unterschiedlichen Kompartimenten davon lokalisiert sind..

Die Herstellung geeigneter "Bakterienghosts" ist beispielsweise in der internationalen Patentanmeldung WO 9 201 791 beschrieben. Dort werden modifizierte Bakterien offenbart, erhältlich durch Transformation eines gram-negativen Bakteriums mit dem Gen eines lytisch wirkenden Membranproteins aus Bakteriophagen, mit dem Gen eines lytisch wirkenden Toxin-Freisetzungsproteins oder mit Genen, die Teilsequenzen davon, die für lytische Proteine kodieren, enthalten, Kultivierung des Bakteriums, Expression dieses Lyse-Gens und Isolierung des resultierenden Bakterienghosts aus dem Kulturmedium.

An die Membran dieser Bakterien kann, wie im europäischen Patent 0 516 655 beschrieben, ein rekombinantes Protein gebunden sein, das durch Expression einer rekombinanten DNA in diesen gram-negativen Bakterien erhältlich ist. Diese rekombinante DNA umfaßt eine erste DNA-Sequenz, welche für eine hydrophobe, nicht lytisch wirkende membranintegrierende Proteindomäne, die eine *α*-helikale Struktur besitzt und aus 14-20 Aminosäuren besteht, die N- und C-terminal von je 2-30 beliebigen Aminosäuren flankiert sein können, kodiert. Mit dieser ersten DNA-Sequenz in operativer Verknüpfung befindet sich eine zweite DNA-Sequenz, die für ein gewünschtes rekombinantes Protein kodiert. Weiterhin enthält das gram-negative Bakterium eine dritte DNA-Sequenz, die unter einer von den ersten und zweiten DNA-Sequenzen getrennten Kontrolle steht und für ein lytisch wirkendes Membranprotein aus Bakteriophagen oder ein lytisch wirkendes Toxin-Freisetzungsprotein oder für deren lytisch wirkende Teile kodiert. Durch Expression und Lyse derartiger rekombinanter, gram-negativer Bakterien werden sog. "Bakterienghosts" erhalten, die eine intakte Oberflächenstruktur mit an die Oberfläche gebundenen immunogenen Epitopen enthalten.

Die Herstellung erfindungsgemäßer Wirtszellen erfolgt vorzugsweise durch ein Verfahren, wobei man
(a) eine Gram-Negative Bakterien wirtszelle bereitstellt, die transformiert ist mit mindestens zwei für verschiedene funktionelle heterologe rekombinante Polypeptide kodierenden Nukleinsäuren, wobei mindestens eine der Nukleinsäuren mit einer Sequenz verknüpft ist, die für ein S-Layer-Struktur Polypeptid kodiert, um eine Expression des heterologen rekombinanten Polypeptids in trägergebundener Form als fusion mit einem S-Layer-Struktur Polypeptid zu ermöglichen, wobei die verschiedenen funktionellen heterologen rekombinanten Polypeptide in jeweils unterschiedlichen Kompartimenten davon lokalisiert sind.
(b) die Wirtszelle unter solchen Bedingungen kultiviert, die zu einer Expression der Nukleinsäuren und zu einer Erzeugung der davon kodierten Polypeptide in funktioneller Form führen.

Die für die rekombinanten Polypeptide kodierenden Nukleinsäuren sind operativ mit Sequenzen verknüpft, die für eine Lokalisierung der rekombinanten Polypeptide in jeweils unterschiedlichen Kompartimenten der Wirtszelle sorgen.

Bei einer Expression der rekombinanten Proteine in trägergebundener Form als modifizierte S-Layer können zusätzlich auch Gene in der Zelle exprimiert werden, die für ein nicht modifiziertes S-Layer-Protein kodieren. In diesem Fall können die modifizierten S-Layer-Proteine eine mit dem nichtmodifizierten S-Layer-Proteine kompatible S-Layer-Struktur ausbilden. Ein Beispiel für diese Ausführungsform des erfindungsgemäßen Verfahrens ist eine E.coli Zelle, die mit vier S-Layer-Genen transformiert ist, von denen zwei natürliche sbsA oder sbsB Gene und die anderen beiden rekombinante sbsA- oder sbsB-Gene sind.

Die für die rekombinanten Polypeptide kodierenden Nukleinsäuren befinden sich vorzugsweise auf rekombinanten Vektoren, die mindestens eine Kopie der Nukleinsäure enthalten. Als Vektoren können übliche prokaryontische oder eukaryontische chromosomale oder extrachromosomale Vektoren eingesetzt werden. Beispiele derartiger Vektoren sind bei Sambrook et al., supra, beschrieben. Die Vektoren enthalten die für die rekombinanten Polypeptide kodierenden Nukleinsäuren in operativer Verknüpfung mit einer in der jeweiligen Wirtszelle aktiven Expressionskontrollsequenz. Besonders bevorzugt umfaßt die Expressionskontrollsequenz einen regulierbaren Promotor. Beispiele für geeignete prokaryontische Promotoren sind der tac-, lac-, trp oder *λ*-Promotor. Beispiele für eukaryontische Promotoren sind der SV40, CMV- oder Metallothionein-Promotor. Zur Expression der mindestens zwei für heterologe Polypeptide kodierenden Nukleinsäuren werden besonders bevorzugt zwei unterschiedliche regulierbare Promotoren verwendet, z.B. zwei unterschiedlich temperatursensitive *λ*-Promotoren wie schon beschrieben.

Die rekombinanten Wirtszellen (lebende Wirtszellen oder Ghosts) sind für eine Vielzahl von Anwendungen geeignet. Bevorzugt ist eine Verwendung als Vakzin oder Adjuvans, wobei man in diesem Fall rekombinante Polypeptide verwendet, die immunogene Epitope von pathogenen und/oder körpereigene immunstimmulierende Polypeptide, wie etwa Cytokine, umfassen.

Besonders bevorzugt ist die Verwendung der erfindungsgemäßen Wirtszellen oder/und Bakterienghosts als Enzymreaktor.

Weiterhin wird die vorliegende Erfindung durch die nachfolgenden Beispiele und Figuren erläutert. Es zeigen:
- SEQ ID NO.1: die vollständige Nukleotidsequenz des kodierenden Abschnitts des S-Layer-Gens sbsA von B.stearothermophilus;
- SEQ ID NO.2: die davon abgeleitete Aminosäuresequenz;
- SEQ ID NO.3: die vollständige Nukleotidsequenz des kodierenden Abschnitts des S-Layer-Gens sbsB von B.stearothermophilus;
- SEQ ID NO.4: die davon abgeleitete Aminosäuresequenz;

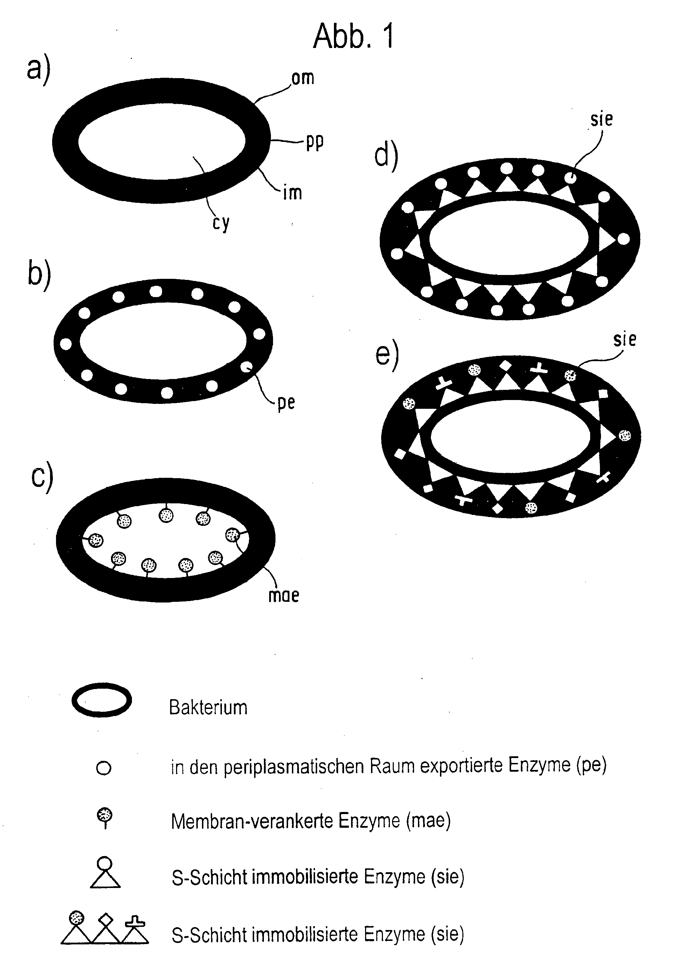

Fig. 1 eine schematische Darstellung über die Möglichkeiten zur Lokalisierung heterologer Polypeptide in unterschiedlichen Kompartimenten einer gram-negativen Bakterienzelle.
(a) Eine gram-negative Bakterienzelle ist aufgebaut aus dem Cytoplasma (cy), der inneren Membran (im), dem Periplasma (pp) und äußeren Membran (om).
(b) Heterologe Polypeptide können durch Verknüpfung mit geeigneten Targetingesequenzen in das Periplasma exportiert werden (pe).
(c) Auf der Innenseite der inneren Membran können heterologe Polypeptide verankert werden (mae).
(d) Im Periplasma können heterologe Polypeptide in Form von rekombinanten S-Layern immobilisiert werden (sie).
(e) nicht nur eine, sondern mehrere Spezies von rekombinanten heterologen Polypeptiden können als S-Layer im Periplasma immobilisiert werden.

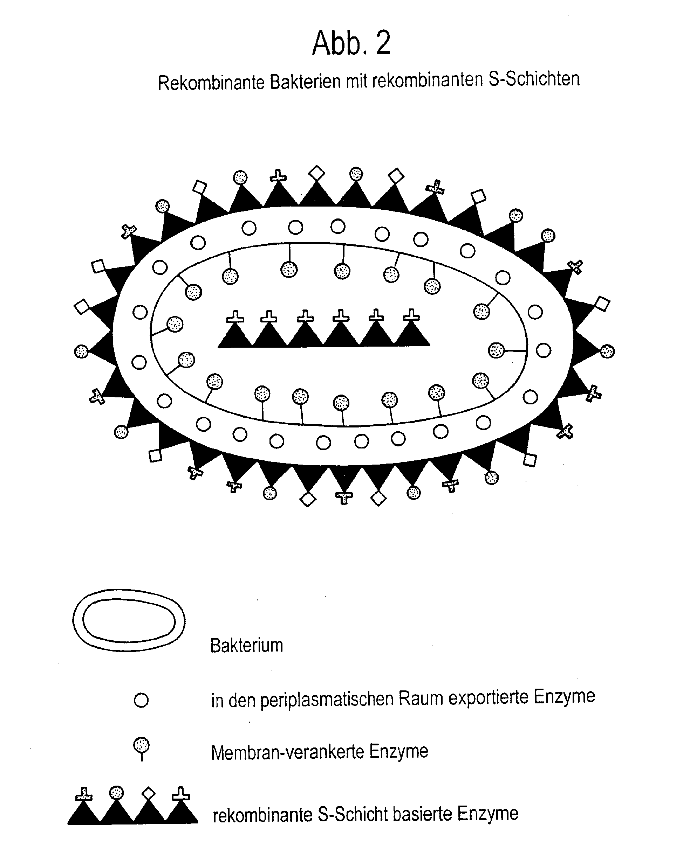

Fig. 2 die schematische Darstellung einer erfindungsgemäßen rekombinanten Bakterienzelle, die verschiedene heterologe Polypeptide (z.B. Enzyme) in unterschiedlichen Kompartimenten enthält.

### BEISPIELE:

### 1. Bakterienstämme, Medien und Plasmide

Gram-positive Bakterien des Stammes Bacillus stearothermophilus PV72 wurden bei 58°C in SVIII-Medium (Bartelmus und Perschak, Z.Zuckerind.7 (1957), 276-281) kultiviert. E.coli Bakterien wurden in LB-Medium kultiviert (Sambrook et al., (1989), supra). Zur Selektion von Transformanten wurde Ampicillin in einer Endkonzentration von 100 *µ*g/ml dem Medium zugegeben. Das Plasmid pPLcAT10 (*λ*pL, bla, colE1) (Stanssens et al., Gene 36 (1985), 211-223) wurde als Klonierungsvektor verwendet.

### 2. Manipulation von DNA-Fragmenten

Restriktionsanalyse von DNA, Agarosegelelektrophorese und Klonierung von DNA-Fragmenten wurden nach den bei Sambrook et al. (1989), supra, beschriebenen Standardmethoden durchgeführt.

Die Transformation von kompetenten Zellen erfolgte durch Elektroporation unter Verwendung eines Bio-Rad Genepulsers (Bio-Rad Laboratories, Richmond, Kalif., USA) nach Protokollen des Herstellers.

Plasmid-DNA wurde nach der Methode von Birnboim und Doly (Nucleic Acids Res.7 (1979), 1513-1523) isoliert. Chromosomale DNA wurde gemäß der bei Ausubel et al. (Current Protocols in Molecular Biology (1987), New York, John Wiley) beschriebenen Verfahren isoliert.

Restriktionsendonukleasen und andere Enzyme wurden von Boehringer Mannheim, New England Biolabs oder Stratagene bezogen und gemäß den Vorschriften der Hersteller eingesetzt.

### 3. DNA-Sequenzierung

Die Sequenzanalyse von DNA-Molekülen erfolgte nach der Didesoxykettenterminationsmethode von Sanger et al.. Die zur Sequenzierung des sbsA-Gens verwendeten Primer wurden auf Basis der bereits publizierten sbsA-Sequenz (Kuen et al., Gene 145 (1994), 115-120) konstruiert.

### 4. PCR-Amplifikation von sbsA

Die PCR-Amplifikation des sbsA-Gens erfolgte gemäß Beispiel 4 von WO 9 906 567.

Die PCR-amplifizierten Produkte wurden auf einem 0.8% Agarosegel elektrophoretisch aufgetrennt und unter Verwendung des Systems von Gene Clean (BIO101 La Jolla, Kalif., USA) zur Klonierung gereinigt.

### 5. Klonierung des sbsA-Gens

### 5.1 Cytoplasmatischer Expressionsvektor

Das durch PCR gewonnene sbsA-Gen mit einer Länge von 3,79 kb wurde gereinigt und mit den Restriktionsendonukleasen Xbal und BamHI gespalten. Das resultierende XbaI-BamHI-Fragment wurde in die entsprechenden Restriktionsstellen des Vektors pPLcAT10 kloniert, so daß das sbsA-Gen unter transkriptioneller Kontrolle des stromaufwärts gelegenen pL-Promotors war. Das ATG-Startkodon der sbsA-Sequenz wurde durch die Klonierungsprozedur rekonstruiert. Die klonierte sbsA-Sequenz enthielt die N-terminale Signalsequenz von sbsA und endete 20 nt nach dem Transkriptionsterminator. Der resultierende Vektor wurde als pBK4 bezeichnet.

### 5.2 Periplasmatischer Expressionsvektor

Das sbsA-Gen wurde ohne Signalsequenz und mit Stopkodon am 3'-Ende in den Polylinker des kommerziell erhältlichen Plasmids pMAL-P2 (New England Biolabs) kloniert. Das resultierende Plasmid pMAL-A enthält unter Kontrolle des tac-Promotors ein Fusionsgen, umfassend das malE-Gen einschließlich dessen Signalsequenz sowie das sbsA-Gen ohne dessen Signalsequenz. Zwischen den beiden Domänen ist eine Faktor Xa Spaltungsstelle angeordnet.

### 6. Rekombinante Coeexpression des sbsA-Gens im Cytoplasma und Periplasma von E.coli

E.coli K12-Zellen cotransformiert mit pBK4 und pMAL-A wurden bei 28°C bis zum Erreichen einer optischen Dichte OD₆₀₀ von 0,3 kultiviert. Dann wurde die cytoplasmatische Expression von sbsA durch Erhöhung der Kultivierungstemperatur von 28°C auf 42°C induziert. Die periplasmatische Expression von sbsA wurde durch Zugabe von 1 mM des lac-Induktors IPTG induziert. 1,5 ml Aliquots wurden vor bzw. 1, 2, 3 und 5 Stunden nach Induktion der sbsA-Expression entnommen. Als Kontrollen wurden E.coli pop2135/pPLcAT10 (kultiviert unter den gleichen Bedingungen) und B.stearothermophilus PV72 verwendet.

Kulturüberstände und Zellextrakte aus allen Proben wurden auf die Expression des S-Layer-Proteins durch SDS-PAGE und Western-Immunoblotting untersucht.

Für den Western Blot wurden die Proteine auf eine Nitrozellulosemembran transferiert und mit einem polyklonalen Antiserum gegen SbsA aus Kaninchen inkubiert. Die Herstellung dieses Antiserums ist bei Egelseer et al. (J.Bacteriol.177 (1995), 1444-1451) beschrieben. Zum Nachweis gebundener SbsA-spezifischer Antikörper wurde ein Konjugat aus Ziegen-Anti-Kaninchen-IgG und alkalischer Phosphatase verwendet.

In cytoplasmatischen Extrakten aus den cotransformierten E.coli-Zellen wurde eine zusätzliche starke Proteinbande mit etwa dem gleichen Molekulargewicht wie das Wildtyp-SbsA-Protein gefunden.

Eine Analyse des Rohextrakts von mit pMAL-A transformierten E.coli DH5*α-*Zellen (Hanahan (1983) supra) zeigte die Expression eines MalE-SbsA Fusionspolypeptids mit einem Molekulargewicht von ca. 170 kDa in der durch eine kalte osmotische Schockprozedur (Neu und Heppel, J. Biol. Chem. 240 (1965); 3685-3692) erzeugten periplasmatischen Fraktion des Zellextrakts.

### 7. Coexpression des SbsB-Proteins im Cytoplasma und Periplasma

Das sbsB-Gen wurde - wie in den Beispielen 5 und 6 beschrieben - in die Plasmide pMAL-P2 und pPLcAT10 kloniert, wobei die Plasmide pMAL-B und pBK6 erhalten wurden.

In mit den Plasmiden pMAL-B und pBK6 transformierten E.coli-Zellen konnte das Vorhandensein des SbsB-Proteins im Cytoplasma und Periplasma nachgewiesen werden.

### 8. Immobilisierte PHB-Synthase in der Cytoplasmamembran von E.coli

Polyhydroxyalkanoate (PHA) sind bakterielle Speicherstoffe, die in einem natürlichen Produzenten bei Limitierung von Phosphor, Schwefel oder Sauerstoff jedoch ausreichend vorhandener C-Quelle gebildet werden. Sie bestehen aus veresterten Monomeren von Hydroxyfettsäuren und bilden wasserunlösliche Polymere, die je nach Stellung der Hydroxylgruppe und der Länge der Seitenketten unterschieden werden. Das prominenteste PHA ist Poly(R)-3-hydroxybutyrat (P(3-HB)) ein unverzweigtes Homopolymer aus (R)-3-Hydroxybuttersäure P(3-HB)).

In dem Gram-negativen, fakultativ chemolithotrophen Knallgasbakterium Ralstonia eutropha sind die für die PHB Synthese verantwortlichen Gene phbA, phbB und phbC in einem Operon (phbCAB) organisiert (Schubert et al., J. Bacteriol (1988), 170:5837). Die Nukleotidsquenzen der offenen Leserahmen sowie der Translationsregionen dieser Gene wurde publiziert (Steinbüchel, A. Polyhydroxy alcanoic acids, in: Biomaterials (1991), 123-213).

Im Rahmen der Untersuchung der Struktur/Funktionsbeziehung der PHB-Synthase wurden verschiedene Insertions- und Deletionsmutanten sowie Fusionsproteine hergestellt und Änderungen in der Enzymaktivität bestimmt (Kalousek, S. Dissertation, Universität Wien (1993)). Aus dieser Arbeit stammt das Plasmid pPHB-L, das ein Gen enthält, das für ein Fusionsprotein aus P(3-HB)-Synthase (588 Aminosäuren von 590) und einem Membrananker aus der C-terminalen Sequenz des Lyseproteins des Phagen MS2 besteht. Zellen, die auf festem Medium + 1 % Glucose wachsen, akkumulieren bei Expression von pPHB-L bis zu 60% (w/w) P(3-HB) Granula.

Die membranverankerte PHB-Synthase ist also enzymatisch voll funktionsfähig.

### 9. Rekombinante Expression der (2,5-Diketo-D-gluconsäure)-Reduktase von einem Plasmid in Bakterien; und Immobilisierung derselben an bakteriellen S-Layers

Durch Expression eines rekombinanten Plasmids in einem Bakterienstamm sollte dieser die Fähigkeit erlangen, aus D-Glucose in einem einzigen Schritt (single-fermentation) direkt 2-KLG herzustellen. Dazu wurde in einen 2,5-Diketo-D-gluconsäure (2,5-DKG) produzierenden Stamm ein für die Reduktion zu 2-KLG notwendiges Enzym (2,5-DKG-Reduktase aus Corynebacterium sp. ATTC. 31090) kloniert.

Durch Verwendung des Vektors pSL, der für ein Surface-Layer Gen (sbsA) kodiert, wurde die Stabilität der 2,5-DKG Reduktase gesteigert. Das S-Layer-Protein (SbsA) diente dabei als Matrix für die 2,5-DKG Reduktase. Durch die Inserton der 2,5-DKG Reduktase an 4 verschiedenen Stellen innerhalb des sbsA Gens wurde ein rekombinantes Protein mit der Fähigkeit, Self-Assembly Produkte zu bilden gefunden.

Hierzu wurden sbsA-2,5 dkg-Reduktase Fusionsgene hergestellt, welche an vier verschiedenen Positionen des sbsA-Gens (Position 581, 881, 916 und 2649) das 2,5-dkg Reduktase Gen tragen. Als Wirtszelle diente E.coli pop2135. Die korrekte Klonierung wurde durch Sequenzieranalysen des insertierten Gens und der angrenzenden Bereiche überprüft. Die stabile Expression des Fusionsproteine konnte durch SDS-PAGE und Western Blot Analysen gezeigt werden. Nach erfolgreicher Expression in E.coli wurden die Plasmide in Pectobacterium cypripedii HaP01 transformiert.

Mittels SDS-PAGE und Western Blot konnte auch in P.cypripedii HaP01 eine stabile Expression des SbsA-2,5 DKG-Reduktase-Fusionsproteins festgestellt werden.

### 10. Weitere an S-Layern immobilisierte Enzyme

An das sbsA Gen wurden auch das Enzym Luziferase (Gene luxA und luxB) und das Green Fluorescent Protein (gfp) fusioniert, um so Licht bzw. Fluoreszenz zu erzeugen.

## Patentansprüche

1. Wirtszelle umfassend mindestens zwei verschiedene funktionelle heterologe rekombinante Polypeptide, von denen mindestens eines in Träger-gebundener Form als Fusion mit einem S-Layer-Struktur Polypeptid vorliegt, wobei die verschiedenen funktionellen heterologen rekombinanten Polypeptide in jeweils unterschiedlichen Kompartimenten davon lokalisiert sind, wobei die Wirtszelle eine gram-negative Bakterienzelle ist.

2. Zelle nach Anspruch 1,
**dadurch gekennzeichnet,**
**dass** die Kompartimente ausgewählt sind aus dem Cytosol, der Außen- und Innenseite der cytoplasmatischen Membran, dem periplasmatischen Raum und der Außen- und Innenseite der Außenmembran.

3. Zelle nach Anspruch 1 oder 2,
**dadurch gekennzeichnet,**
**dass** mehrere der rekombinanten Polypeptide in Träger-gebundener Form vorliegen.

4. Zelle nach einem der Ansprüche 1 bis 3,
**dadurch gekennzeichnet,**
**dass** weitere Träger-gebundene Polypeptide in Form von S-Layer-Strukturen, Membran-gebundenen Polypeptiden oder/und als Bestandteile rekombinanter Phagenstrukturen vorliegen.

5. Wirtszelle nach einem der Ansprüche 1 bis 4,
**dadurch gekennzeichnet,**
**dass** die rekombinanten Polypeptide ausgewählt sind aus Enzymen, Cytokinen, Antikörper-bindenden Proteinen, DNA-bindenden Epitopen, antigenen, allergenen und immunogenen Epitopen und Strepavidin.

6. Zelle nach einem der Ansprüche 1 bis 5,
**dadurch gekennzeichnet,**
**dass** die rekombinanten Polypeptide Enzyme sind.

7. Wirtszelle nach Anspruch 6,
**dadurch gekennzeichnet,**
**dass** die Enzyme eine mehrstufige enzymatische Reaktion katalysieren.

8. Wirtszelle nach Anspruch 6 oder 7,
**dadurch gekennzeichnet,**
**dass** die Enzyme die Synthese von Polyhydroxyalkanoaten katalysieren.

9. Wirtszelle nach Anspruch 1, wobei die mindestens zwei verschiedenen funktionellen heterologen rekombinanten Polypeptide eine gemeinsame immunologische und/oder biologische Funktion erfüllen.

10. Rekombinanter Bakterienghost, erhältlich aus einer gram-negativen Zelle, umfassend mindestens zwei verschiedene funktionelle heterologe rekombinante Polypeptide, von denen mindestens eines in Träger-gebundener Form als Fusion mit einem S-Layer Struktur Polypeptid vorliegt, wobei die verschiedenen funktionellen heterologen rekombinanten Polypeptide in jeweils unterschiedlichen Kompartimenten davon lokalisiert sind.

11. Rekombinanter Bakterienghost nach Anspruch 10, wobei die mindestens zwei verschiedenen funktionellen heterologen rekombinanten Polypeptide eine gemeinsame immunologische und/oder biologische Funktion erfüllen.

12. Verfahren zur Herstellung einer Wirtszelle nach einem der vorhergehenden Ansprüche 1 bis 9,
**dadurch gekennzeichnet,**
**dass** man
(a) eine Wirtszelle bereitstellt, die transformiert ist mit mindestens zwei für verschiedene funktionelle heterologe rekombinante Polypeptide codierenden Nukleinsäuren, wobei mindestens eine der Nukleinsäuren mit einer Sequenz verknüpft ist, die für ein S-Layer Struktur Polypeptid codiert, um eine Expression des heterologen rekombinanten Polypeptids in Träger-gebundener Form als Fusion mit einem S-Layer Struktur Polypeptid zu ermöglichen, wobei die Polypeptide in jeweils unterschiedlichen Kompartimenten davon lokalisiert sind, wobei die Wirtszelle eine gram-negative Bakterienzelle ist,
(b) die Wirtszelle unter solchen Bedingungen kultiviert, die zu einer Expression der Nukleinsäuren und zu einer Erzeugung der davon codierten Polypeptide in funktioneller Form führen.

13. Verwendung einer Zelle nach einem der Ansprüche 1 bis 9 oder eines Ghosts nach Anspruch 10 oder 11 zur Herstellung eines Vakzins oder Adjuvans.

14. Verwendung einer Wirtszelle nach einem der Ansprüche 1 bis 9 oder eines Ghosts nach Anspruch 10 oder 11 als Enzymreaktor.

## Claims

1. Host cell comprising at least two differrent functional heterologous recombinant polypeptides, at least one of which is present in a support-bound form as a fusion with an S-layer-structure polypeptide, wherein the different functional heterologous recombinant polypeptides are located in each case in different compartments thereof, wherein the host cell is a gram-negative bacterial cell.

2. Cell according to claim 1, **characterised in that** the compartments are selected from among cytosol, the outside and inside of the cytoplasmic membrane, the periplasmic space and the outside and inside of the outer membrane.

3. Cell according to either claim 1 or claim 2, **characterised in that** a plurality of the recombinant polypeptides are present in a support-bound form.

4. Cell according to any of claims 1 to 3, **characterised in that** additional support-bound polypeptides are present in the form of S-layer structures, membrane-bound polypeptides and/or as components of recombinant phage structures.

5. Host cell according to any of claims 1 to 4, **characterised in that** the recombinant polypeptides are selected from among enzymes, cytokines, antibody-binding proteins, DNA-binding epitopes, antigenic, allergenic and immunogenic epitopes and streptavidin.

6. Cell according to any of claims 1 to 5, **characterised in that** the recombinant polypeptides are enzymes.

7. Host cell according to claim 6, **characterised in that** the enzymes catalyse a multistage enzymatic reaction.

8. Host cell according to either claim 6 or claim 7, **characterised in that** the enzymes catalyse the synthesis of polyhydroxyalkanoates.

9. Host cell according to claim 1, wherein the at least two different functional heterologous recombinant polypeptides perform a common immunological and/or biological function.

10. Recombinant bacterial ghost obtainable from a gram-negative cell, comprising at least two different functional heterologous recombinant polypeptides, at least one of which is present in a support-bound form as a fusion with an S-layer-structure polypeptide, wherein the different functional heterologous recombinant polypeptides are located in each case in different compartments thereof.

11. Recombinant bacterial ghost according to claim 10, wherein the at least two different functional heterologous recombinant polypeptides perform a common immunological and/or biological function.

12. Method for preparing a host cell according to any of claims 1 to 9, **characterised in that**
(a) a host cell is provided which is transformed with at least two nucleic acids encoding different functional heterologous recombinant polypeptides, at least one of the nucleic acids being linked to a sequence encoding an S-layer-structure polypeptide in order to facilitate expression of the heterologous recombinant polypeptide in a support-bound form as a fusion with an S-layer-structure polypeptide, the polypeptides being located in each case in different compartments thereof, the host cell being a gram-negative bacterial cell,
(b) the host cell is cultured under such conditions which induce expression of the nucleic acids and generation of the polypeptides, encoded thereby, in a functional form.

13. Use of a cell according to any of claims 1 to 9 or of a ghost according to either claim 10 or claim 11 to prepare a vaccine or an adjuvant.

14. Use of a host cell according to any of claims 1 to 9 or of a ghost according to either claim 10 or claim 11 as an enzyme reactor.

## Revendications

1. Cellule hôte comprenant au moins deux polypeptides recombinés hétérologues fonctionnels différents, dont au moins un est présent sous une forme liée à un support en tant que fusion avec un polypeptide de structure S-Layer, où les différents polypeptides recombinés hétérologues fonctionnels sont localisés dans des compartiments différents de celle-ci, où la cellule hôte est une cellule bactérienne Gram-négative.

2. Cellule selon la revendication 1, **caractérisée en ce que** les compartiments sont choisis parmi le cytosol, les faces extérieure et intérieure de la membrane cytoplasmique, l'espace périplasmique et les faces extérieure et intérieure de la membrane externe.

3. Cellule selon la revendication 1 ou 2, **caractérisée en ce que** plusieurs des polypeptides recombinés sont présents sous une forme liée à un support.

4. Cellule selon l'une des revendications 1 à 3, **caractérisée en ce que** d'autres polypeptides liés à un support sont présents sous forme de structures S-Layer, de polypeptides liés à une membrane et/ou comme constituant de structures de phage recombiné.

5. Cellule selon l'une des revendications 1 à 4, **caractérisée en ce que** les polypeptides recombinés sont choisis parmi des enzymes, des cytokines, des protéines liant les anticorps, des épitopes liant l'ADN, des épitopes antigènes, allergènes et immunogènes et la streptavidine.

6. Cellule selon l'une des revendications 1 à 5, **caractérisée en ce que** le polypeptide recombiné est une enzyme.

7. Cellule hôte selon la revendication 6, **caractérisée en ce que** l'enzyme catalyse une réaction enzymatique à plusieurs étapes.

8. Cellule hôte selon la revendication 6 ou 7, **caractérisée en ce que** l'enzyme catalyse la synthèse des polyhydroxyalcanoates.

9. Cellule hôte selon la revendication 1, où les au moins deux polypeptides recombinés hétérologues fonctionnels différents remplissent une fonction immunologique et/ou biologique commune.

10. Fantôme bactérien recombiné, obtenu à partir d'une cellule Gram-négative, comprenant au moins deux polypeptides recombinés hétérologues fonctionnels différents, dont au moins un est présent sous une forme liée à un support en tant que fusion avec un polypeptide de structure S-layer, où les différents polypeptides recombinés hétérologues fonctionnels sont localisés dans des compartiments différents de celle-ci.

11. Fantôme bactérien recombiné selon la revendication 10, où les au moins deux polypeptides recombinés hétérologues fonctionnels différents remplissent une fonction immunologique et/ou biologique commune.

12. Procédé de préparation d'une cellule hôte selon l'une des revendications 1 à 9 précédentes, **caractérisé en ce que**
(a) on prépare une cellule hôte qui est transformée avec au moins deux acides nucléiques codant des polypeptides recombinés hétérologues fonctionnels différents, où au moins un des acides nucléiques est relié à une séquence qui code un polypeptide à structure S-Layer pour permettre l'expression du polypeptide hétérologue recombiné sous une forme liée à un support en tant que fusion avec un polypeptide de structure S-Layer, où les polypeptides sont localisés dans des compartiments différents de celle-ci, où la cellule hôte est une cellule bactérienne Gram-négative,
(b) la cellule hôte est mise en culture dans des conditions qui conduisent à l'expression des acides nucléiques et à la production des polypeptides codés par ceux-ci sous forme fonctionnelle.

13. Utilisation d'une cellule selon l'une des revendications 1 à 9 ou d'un fantôme selon la revendication 10 ou 11, pour la préparation d'un vaccin ou d'un adjuvant.

14. Utilisation d'une cellule hôte selon l'une des revendications 1 à 9 ou d'un fantôme selon la revendication 10 ou 11, comme réacteur enzymatique.


### SEQUENZPROTOKOLL

(1) ALLGEMEINE ANGABEN:
   (i) ANMELDER:
      (A) NAME: Werner Lubitz
      (B) STRASSE: Schoenborngasse 12/7
      (C) ORT: Wien
      (E) LAND: Austria
      (F) POSTLEITZAHL: 1080
   (ii) BEZEICHNUNG DER ERFINDUNG: Sekretion von S-Layer-Proteinen in das Periplasma und in den extrazellulären Raum
   (iii) ANZAHL DER SEQUENZEN: 6
   (iv) COMPUTER-LESBARE FASSUNG: (A) DATENTRÄGER: Floppy disk (B) COMPUTER: IBM PC compatible (C) BETRIEBSSYSTEM: PC-DOS/MS-DOS (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 3687 Basenpaare
      (B) ART: Nucleotid
      (C) STRANGFORM: beides
      (D) TOPOLOGIE: linear
   (vi) URSPRÜNGLICHE HERKUNFT: (A) ORGANISMUS: Bacillus stearothermophilus (B) STAMM: PV72
   (vii) UNMITTELBARE HERKUNFT:
      (B) CLON(E): sbsA
   (ix) MERKMAL:
      (A) NAME/SCHLÜSSEL: CDS
      (B) LAGE:1..3684
   (ix) MERKMAL:
      (A) NAME/SCHLÜSSEL: sig_peptide
      (B) LAGE:1..90
   (ix) MERKMAL:
      (A) NAME/SCHLÜSSEL: mat peptide
      (B) LAGE:91..3684
   (xi) SEQUENZBESCHREIBUNG: SEQ ID NO: 1:
   ATG GAT AGG AAA AAA GCT GTG AAA CTA GCA ACA GCA AGT GCT ATT GCA       48
   Met Asp Arg Lys Lys Ala Val Lys Leu Ala Thr Ala Ser Ala Ile Ala
   -30                 -25                 -20                 -15
   GCA AGT GCA TTT GTC GCT GCA AAT CCA AAC GCT TCT GAA GCG GCT ACA       96
   Ala Ser Ala Phe Val Ala Ala Asn Pro Asn Ala Ser Glu Ala Ala Thr
                   -10                  -5                   1
   GAT GTA GCA ACA GTA GTA AGC CAA GCA AAA GCA CAG TTC AAA AAA GCA      144
   Asp Val Ala Thr Val Val Ser Gln Ala Lys Ala Gln Phe Lys Lys Ala
               5                  10                  15
   TAC TAT ACT TAC AGC CAT ACA GTA ACG GAA ACT GGT GAA TTC CCA AAC      192
   Tyr Tyr Thr Tyr Ser His Thr Val Thr Glu Thr Gly Glu Phe Pro Asn
       20                  25                  30
   ATT AAC GAT GTA TAT GCT GAA TAC AAC AAA GCG AAA AAA CGA TAC CGT      240
   Ile Asn Asp Val Tyr Ala Glu Tyr Asn Lys Ala Lys Lys Arg Tyr Arg
   35                  40                  45                  50
   GAT GCG GTA GCA TTA GTG AAT AAA GCA GGT GGC GCG AAA AAA GAC GCT      288
   Asp Ala Val Ala Leu Val Asn Lys Ala Gly Gly Ala Lys Lys Asp Ala
                   55                  60                  65
   TAC TTA GCT GAT TTA CAA AAA GAA TAT GAA ACT TAC GTT TTC AAA GCA      336
   Tyr Leu Ala Asp Leu Gln Lys Glu Tyr Glu Thr Tyr Val Phe Lys Ala
               70                  75                  80
   AAC CCT AAA TCT GGC GAA GCT CGT GTA GCA ACT TAC ATC GAT GCT TAC      384
   Asn Pro Lys Ser Gly Glu Ala Arg Val Ala Thr Tyr Ile Asp Ala Tyr
           85                  90                  95
   AAC TAT GCA ACA AAA TTA GAC GAA ATG CGC CAA GAG CTA GAG GCT GCT      432
   Asn Tyr Ala Thr Lys Leu Asp Glu Met Arg Gln Glu Leu Glu Ala Ala
       100                 105                 110
   GTT CAA GCA AAA GAT TTA GAA AAA GCA GAA CAA TAC TAT CAC AAA ATT      480
   Val Gln Ala Lys Asp Leu Glu Lys Ala Glu Gln Tyr Tyr His Lys Ile
   115                 120                 125                 130
   CCT TAT GAA ATT AAA ACT CGC ACA GTC ATT TTA GAT CGC GTA TAT GGT      528
   Pro Tyr Glu Ile Lys Thr Arg Thr Val Ile Leu Asp Arg Val Tyr Gly
                   135                 140                 145
   AAA ACA ACT CGT GAT TTA CTT CGC TCT ACA TTT AAA GCA AAA GCA CAA      576
   Lys Thr Thr Arg Asp Leu Leu Arg Ser Thr Phe Lys Ala Lys Ala Gln
               150                 155                 160
   GAA CTT CGC GAC AGC TTA ATT TAT GAT ATT ACC GTT GCA ATG AAA GCG      624
   Glu Leu Arg Asp Ser Leu Ile Tyr Asp Ile Thr Val Ala Met Lys Ala
           165                 170                 175
   CGC GAA GTA CAA GAC GCT GTG AAA GCA GGC AAT TTA GAC AAA GCT AAA      672
   Arg Glu Val Gln Asp Ala Val Lys Ala Gly Asn Leu Asp Lys Ala Lys
       180                 185                 190
   GCT GCT GTT GAT CAA ATC AAT CAA TAC TTA CCA AAA GTA ACA GAT GCT      720
   Ala Ala Val Asp Gln Ile Asn Gln Tyr Leu Pro Lys Val Thr Asp Ala
   195                 200                 205                 210
   TTC AAA ACT GAA CTA ACA GAA GTA GCG AAA AAA GCA TTA GAT GCA GAT      768
   Phe Lys Thr Glu Leu Thr Glu Val Ala Lys Lys Ala Leu Asp Ala Asp
                   215                 220                 225
   GAA GCT GCG CTT ACT CCA AAA GTT GAA AGT GTA AGT GCG ATT AAC ACT      816
   Glu Ala Ala Leu Thr Pro Lys Val Glu Ser Val Ser Ala Ile Asn Thr
               230                 235                 240
   CAA AAC AAA GCT GTT GAA TTA ACA GCA GTA CCA GTG AAC GGA ACA CTA      864
   Gln Asn Lys Ala Val Glu Leu Thr Ala Val Pro Val Asn Gly Thr Leu
           245                 250                 255
   AAA TTA CAA CTT TCA GCT GCT GCA AAT GAA GAT ACA GTA AAC GTA AAT      912
   Lys Leu Gln Leu Ser Ala Ala Ala Asn Glu Asp Thr Val Asn Val Asn
       260                 265                 270
   ACT GTA CGT ATC TAT AAA GTG GAC GGT AAC ATT CCA TTT GCC CTT AAT      960
   Thr Val Arg Ile Tyr Lys Val Asp Gly Asn Ile Pro Phe Ala Leu Asn
   275                 280                 285                 290
   ACG GCA GAT GTT TCT TTA TCT ACA GAC GGA AAA ACT ATC ACT GTG GAT     1008
   Thr Ala Asp Val Ser Leu Ser Thr Asp Gly Lys Thr Ile Thr Val Asp
                   295                 300                 305
   GCT TCA ACT CCA TTC GAA AAT AAT ACG GAG TAT AAA GTA GTA GTT AAA     1056
   Ala Ser Thr Pro Phe Glu Asn Asn Thr Glu Tyr Lys Val Val Val Lys
               310                 315                 320
   GGT ATT AAA GAC AAA AAT GGC AAA GAA TTT AAA GAA GAT GCA TTC ACT     1104
   Gly Ile Lys Asp Lys Asn Gly Lys Glu Phe Lys Glu Asp Ala Phe Thr
           325                 330                 335
   TTC AAG CTT CGA AAT GAT GCT GTA GTT ACT CAA GTG TTT GGA ACT AAT     1152
   Phe Lys Leu Arg Asn Asp Ala Val Val Thr Gln Val Phe Gly Thr Asn
       340                 345                 350
   GTA ACA AAC AAC ACT TCT GTA AAC TTA GCA GCA GGT ACT TTC GAC ACT     1200
   Val Thr Asn Asn Thr Ser Val Asn Leu Ala Ala Gly Thr Phe Asp Thr
   355                 360                 365                 370
   GAC GAT ACT TTA ACA GTA GTA TTT GAT AAG TTG TTA GCA CCT GAA ACT     1248
   Asp Asp Thr Leu Thr Val Val Phe Asp Lys Leu Leu Ala Pro Glu Thr
                   375                 380                 385
   GTA AAC AGC TCG AAC GTT ACT ATT ACA GAT GTT GAA ACT GGA AAA CGC     1296
   Val Asn Ser Ser Asn Val Thr Ile Thr Asp Val Glu Thr Gly Lys Arg
               390                 395                 400
   ATT CCA GTA ATT GCA TCT ACT TCT GGT TCT ACA ATT ACT ATT ACG TTA     1344
   Ile Pro Val Ile Ala Ser Thr Ser Gly Ser Thr Ile Thr Ile Thr Leu
           405                 410                 415
   AAA GAA GCG TTA GTA ACT GGT AAA CAA TAT AAA CTT GCT ATC AAT AAT     1392
   Lys Glu Ala Leu Val Thr Gly Lys Gln Tyr Lys Leu Ala Ile Asn Asn
       420                 425                 430
   GTT AAA ACA TTA ACT GGT TAC AAT GCA GAA GCT TAC GAG TTA GTG TTC     1440
   Val Lys Thr Leu Thr Gly Tyr Asn Ala Glu Ala Tyr Glu Leu Val Phe
   435                 440                 445                 450
   ACT GCA AAC GCA TCA GCA CCA ACT GTT GCT ACC GCT CCT ACT ACT TTA     1488
   Thr Ala Asn Ala Ser Ala Pro Thr Val Ala Thr Ala Pro Thr Thr Leu
                   455                 460                 465
   GGT GGT ACA ACT TTA TCT ACT GGT TCT CTT ACA ACA AAT GTT TGG GGT     1536
   Gly Gly Thr Thr Leu Ser Thr Gly Ser Leu Thr Thr Asn Val Trp Gly
               470                 475                 480
   AAA TTG GCT GGT GGT GTG AAT GAA GCT GGA ACT TAT TAT CCT GGT CTT      1584
   Lys Leu Ala Gly Gly Val Asn Glu Ala Gly Thr Tyr Tyr Pro Gly Leu
           485                 490                 495
   CAA TTC ACA ACA ACG TTT GCT ACT AAG TTA GAC GAA TCT ACT TTA GCT      1632
   Gln Phe Thr Thr Thr Phe Ala Thr Lys Leu Asp Glu Ser Thr Leu Ala
       500                 505                 510
   GAT AAC TTT GTA TTA GTT GAA AAA GAA TCT GGT ACA GTT GTT GCT TCT      1680
   Asp Asn Phe Val Leu Val Glu Lys Glu Ser Gly Thr Val Val Ala Ser
   515                 520                 525                 530
   GAA CTA AAA TAT AAT GCA GAC GCT AAA ATG GTA ACT TTA GTG CCA AAA      1728
   Glu Leu Lys Tyr Asn Ala Asp Ala Lys Met Val Thr Leu Val Pro Lys
                   535                 540                 545
   GCG GAC CTT AAA GAA AAT ACA ATC TAT CAA ATC AAA ATT AAA AAA GGC      1776
   Ala Asp Leu Lys Glu Asn Thr Ile Tyr Gln Ile Lys Ile Lys Lys Gly
               550                 555                 560
   TTG AAG TCC GAT AAA GGT ATT GAA TTA GGC ACT GTT AAC GAG AAA ACA      1824
   Leu Lys Ser Asp Lys Gly Ile Glu Leu Gly Thr Val Asn Glu Lys Thr
           565                 570                 575
   TAT GAG TTC AAA ACT CAA GAC TTA ACT GCT CCT ACA GTT ATT AGC GTA      1872
   Tyr Glu Phe Lys Thr Gln Asp Leu Thr Ala Pro Thr Val Ile Ser Val
       580                 585                 590
   ACG TCT AAA AAT GGC GAC GCT GGA TTA AAA GTA ACT GAA GCT CAA GAA      1920
   Thr Ser Lys Asn Gly Asp Ala Gly Leu Lys Val Thr Glu Ala Gln Glu
   595                 600                 605                 610
   TTT ACT GTG AAG TTC TCA GAG AAT TTA AAT ACA TTT AAT GCT ACA ACC      1968
   Phe Thr Val Lys Phe Ser Glu Asn Leu Asn Thr Phe Asn Ala Thr Thr
                   615                 620                 625
   GTT TCG GGT AGC ACA ATC ACA TAC GGT CAA GTT GCT GTA GTA AAA GCG      2016
   Val Ser Gly Ser Thr Ile Thr Tyr Gly Gln Val Ala Val Val Lys Ala
               630                 635                 640
   GGT GCA AAC TTA TCT GCT CTT ACA GCA AGT GAC ATC ATT CCA GCT AGT      2064
   Gly Ala Asn Leu Ser Ala Leu Thr Ala Ser Asp Ile Ile Pro Ala Ser
           645                 650                 655
   GTT GAA GCG GTT ACT GGT CAA GAT GGA ACA TAC AAA GTG AAA GTT GCT      2112
   Val Glu Ala Val Thr Gly Gln Asp Gly Thr Tyr Lys Val Lys Val Ala
       660                 665                 670
   GCT AAC CAA TTA GAA CGT AAC CAA GGG TAC AAA TTA GTA GTG TTC GGT      2160
   Ala Asn Gln Leu Glu Arg Asn Gln Gly Tyr Lys Leu Val Val Phe Gly
   675                 680                 685                 690
   AAA GGT GCA ACA GCT CCT GTT AAA GAT GCT GCA AAT GCA AAT ACT TTA      2208
   Lys Gly Ala Thr Ala Pro Val Lys Asp Ala Ala Asn Ala Asn Thr Leu
                   695                 700                 705
   GCA ACT AAC TAT ATC TAT ACA TTT ACA ACT GAA GGT CAA GAC GTA ACA      2256
   Ala Thr Asn Tyr Ile Tyr Thr Phe Thr Thr Glu Gly Gln Asp Val Thr
               710                 715                 720
   GCA CCA ACG GTT ACA AAA GTA TTC AAA GGT GAT TCT TTA AAA GAC GCT      2304
   Ala Pro Thr Val Thr Lys Val Phe Lys Gly Asp Ser Leu Lys Asp Ala
           725                 730                 735
   GAT GCA GTT ACT ACA CTT ACG AAC GTT GAT GCA GGT CAA AAA TTC ACT      2352
   Asp Ala Val Thr Thr Leu Thr Asn Val Asp Ala Gly Gln Lys Phe Thr
       740                 745                 750
   ATC CAA TTT AGC GAA GAA TTA AAA ACT TCT AGT GGT TCT TTA GTG GGT     2400
   Ile Gln Phe Ser Glu Glu Leu Lys Thr Ser Ser Gly Ser Leu Val Gly
   755                 760                 765                 770
   GGC AAA GTA ACT GTC GAG AAA TTA ACA AAC AAC GGA TGG GTA GAT GCT     2448
   Gly Lys Val Thr Val Glu Lys Leu Thr Asn Asn Gly Trp Val Asp Ala
                   775                 780                 785
   GGT ACT GGA ACA ACT GTA TCA GTT GCT CCT AAG ACA GAT GCA AAT GGT     2496
   Gly Thr Gly Thr Thr Val Ser Val Ala Pro Lys Thr Asp Ala Asn Gly
               790                 795                 800
   AAA GTA ACA GCT GCT GTG GTT ACA TTA ACT GGT CTT GAC AAT AAC GAC     2544
   Lys Val Thr Ala Ala Val Val Thr Leu Thr Gly Leu Asp Asn Asn Asp
           805                 810                 815
   AAA GAT GCG AAA TTG CGT CTG GTA GTA GAT AAG TCT TCT ACT GAT GGA     2592
   Lys Asp Ala Lys Leu Arg Leu Val Val Asp Lys Ser Ser Thr Asp Gly
       820                 825                 830
   ATT GCT GAT GTA GCT GGT AAT GTA ATT AAG GAA AAA GAT ATT TTA ATT     2640
   Ile Ala Asp Val Ala Gly Asn Val Ile Lys Glu Lys Asp Ile Leu Ile
   835                 840                 845                 850
   CGT TAC AAC AGC TGG AGA CAC ACT GTA GCT TCT GTG AAA GCT GCT GCT     2688
   Arg Tyr Asn Ser Trp Arg His Thr Val Ala Ser Val Lys Ala Ala Ala
                   855                 860                 865
   GAC AAA GAT GGT CAA AAC GCT TCT GCT GCA TTC CCA ACA AGC ACT GCA     2736
   Asp Lys Asp Gly Gln Asn Ala Ser Ala Ala Phe Pro Thr Ser Thr Ala
               870                 875                 880
   ATT GAT ACA ACT AAG AGC TTA TTA GTT GAA TTC AAT GAA ACT GAT TTA     2784
   Ile Asp Thr Thr Lys Ser Leu Leu Val Glu Phe Asn Glu Thr Asp Leu
           885                 890                 895
   GCG GAA GTT AAA CCT GAG AAC ATC GTT GTT AAA GAT GCA GCA GGT AAT     2832
   Ala Glu Val Lys Pro Glu Asn Ile Val Val Lys Asp Ala Ala Gly Asn
       900                 905                 910
   GCG GTA GCT GGT ACT GTA ACA GCA TTA GAC GGT TCT ACA AAT AAA TTT     2880
   Ala Val Ala Gly Thr Val Thr Ala Leu Asp Gly Ser Thr Asn Lys Phe
   915                 920                 925                 930
   GTA TTC ACT CCA TCT CAA GAA TTA AAA GCT GGT ACA GTT TAC TCT GTA     2928
   Val Phe Thr Pro Ser Gln Glu Leu Lys Ala Gly Thr Val Tyr Ser Val
                   935                 940                 945
   ACA ATT GAC GGT GTG AGA GAT AAA GTA GGT AAC ACA ATC TCT AAA TAC     2976
   Thr Ile Asp Gly Val Arg Asp Lys Val Gly Asn Thr Ile Ser Lys Tyr
               950                 955                 960
   ATT ACT TCG TTC AAG ACT GTA TCT GCG AAT CCA ACG TTA TCT TCA ATC     3024
   Ile Thr Ser Phe Lys Thr Val Ser Ala Asn Pro Thr Leu Ser Ser Ile
           965                 970                 975
   AGC ATT GCT GAC GGT GCA GTT AAC GTT GAC CGT TCT AAA ACA ATT ACA     3072
   Ser Ile Ala Asp Gly Ala Val Asn Val Asp Arg Ser Lys Thr Ile Thr
       980                 985                 990
   ATT GAA TTC AGC GAT TCA GTT CCA AAC CCA ACA ATC ACT CTT AAG AAG     3120
   Ile Glu Phe Ser Asp Ser Val Pro Asn Pro Thr Ile Thr Leu Lys Lys
   995                 1000                1005                1010
   GCT GAC GGA ACT TCA TTT ACT AAT TAC ACT TTA GTA AAT GTA AAT AAT     3168
   Ala Asp Gly Thr Ser Phe Thr Asn Tyr Thr Leu Val Asn Val Asn Asn
                   1015                1020                1025
   GAA AAT AAA ACA TAC AAA ATT GTA TTC CAC AAA GGT GTA ACA CTT GAC      3216
   Glu Asn Lys Thr Tyr Lys Ile Val Phe His Lys Gly Val Thr Leu Asp
               1030                1035                1040
   GAG TTT ACT CAA TAT GAG TTA GCA GTT TCA AAA GAT TTT CAA ACT GGT      3264
   Glu Phe Thr Gln Tyr Glu Leu Ala Val Ser Lys Asp Phe Gln Thr Gly
           1045                1050                1055
   ACT GAT ATT GAT AGC AAA GTT ACA TTC ATC ACA GGT TCT GTT GCT ACT      3312
   Thr Asp Ile Asp Ser Lys Val Thr Phe Ile Thr Gly Ser Val Ala Thr
       1060                1065                1070
   GAC GAA GTA AAA CCT GCT CTA GTA GGC GTT GGT TCA TGG AAT GGA ACA      3360
   Asp Glu Val Lys Pro Ala Leu Val Gly Val Gly Ser Trp Asn Gly Thr
   1075                1080                1085                1090
   AGC TAT ACT CAG GAT GCT GCA GCA ACA CGA CTT CGG TCT GTA GCT GAC      3408
   Ser Tyr Thr Gln Asp Ala Ala Ala Thr Arg Leu Arg Ser Val Ala Asp
                   1095                1100                1105
   TTC GTT GCG GAG CCA GTT GCC CTT CAA TTC TCA GAA GGT ATC GAT TTA      3456
   Phe Val Ala Glu Pro Val Ala Leu Gln Phe Ser Glu Gly Ile Asp Leu
               1110                1115                1120
   ACG AAT GCA ACT GTG ACA GTA ACA AAT ATT ACT GAT GAT AAA ACT GTT      3504
   Thr Asn Ala Thr Val Thr Val Thr Asn Ile Thr Asp Asp Lys Thr Val
           1125                1130                1135
   GAA GTT ATT TCA AAA GAG AGT GTA GAC GCA GAC CAT GAT GCA GGT GCT      3552
   Glu Val Ile Ser Lys Glu Ser Val Asp Ala Asp His Asp Ala Gly Ala
       1140                1145                1150
   ACT AAG GAG ACA TTA GTA ATT AAC ACA GTT ACT CCT TTA GTA CTT GAT      3600
   Thr Lys Glu Thr Leu Val Ile Asn Thr Val Thr Pro Leu Val Leu Asp
   1155                1160                1165                1170
   AAC AGC AAG ACT TAT AAG ATT GTT GTA AGT GGA GTT AAA GAT GCA GCA      3648
   Asn Ser Lys Thr Tyr Lys Ile Val Val Ser Gly Val Lys Asp Ala Ala
                   1175                1180                1185
   GGT AAT GTT GCA GAT ACT ATT ACA TTC TAT ATT AAG TAA                  3687
   Gly Asn Val Ala Asp Thr Ile Thr Phe Tyr Ile Lys
               1190                1195
(2) ANGABEN ZU SEQ ID NO: 2:
   (i) SEQUENZKENNZEICHEN:
      (A) LÄNGE: 1228 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Asp Arg Lys Lys Ala Val Lys Leu Ala Thr Ala Ser Ala Ile Ala
   -30                 -25                 -20                 -15
   Ala Ser Ala Phe Val Ala Ala Asn Pro Asn Ala Ser Glu Ala Ala Thr
                   -10                 -5                   1
   Asp Val Ala Thr Val Val Ser Gln Ala Lys Ala Gln Phe Lys Lys Ala
               5                   10                  15
   Tyr Tyr Thr Tyr Ser His Thr Val Thr Glu Thr Gly Glu Phe Pro Asn
       20                  25                  30
   Ile Asn Asp Val Tyr Ala Glu Tyr Asn Lys Ala Lys Lys Arg Tyr Arg
   35                  40                  45                  50
   Asp Ala Val Ala Leu Val Asn Lys Ala Gly Gly Ala Lys Lys Asp Ala
                   55                  60                  65
   Tyr Leu Ala Asp Leu Gln Lys Glu Tyr Glu Thr Tyr Val Phe Lys Ala
               70                  75                  80
   Asn Pro Lys Ser Gly Glu Ala Arg Val Ala Thr Tyr Ile Asp Ala Tyr
           85                  90                  95
   Asn Tyr Ala Thr Lys Leu Asp Glu Met Arg Gln Glu Leu Glu Ala Ala
       100                 105                 110
   Val Gln Ala Lys Asp Leu Glu Lys Ala Glu Gln Tyr Tyr His Lys Ile
   115                 120                 125                 130
   Pro Tyr Glu Ile Lys Thr Arg Thr Val Ile Leu Asp Arg Val Tyr Gly
                   135                 140                 145
   Lys Thr Thr Arg Asp Leu Leu Arg Ser Thr Phe Lys Ala Lys Ala Gln
               150                 155                 160
   Glu Leu Arg Asp Ser Leu Ile Tyr Asp Ile Thr Val Ala Met Lys Ala
           165                 170                 175
   Arg Glu Val Gln Asp Ala Val Lys Ala Gly Asn Leu Asp Lys Ala Lys
       180                 185                 190
   Ala Ala Val Asp Gln Ile Asn Gln Tyr Leu Pro Lys Val Thr Asp Ala
   195                 200                 205                 210
   Phe Lys Thr Glu Leu Thr Glu Val Ala Lys Lys Ala Leu Asp Ala Asp
                   215                 220                 225
   Glu Ala Ala Leu Thr Pro Lys Val Glu Ser Val Ser Ala Ile Asn Thr
               230                 235                 240
   Gln Asn Lys Ala Val Glu Leu Thr Ala Val Pro Val Asn Gly Thr Leu
           245                 250                 255
   Lys Leu Gln Leu Ser Ala Ala Ala Asn Glu Asp Thr Val Asn Val Asn
       260                 265                 270
   Thr Val Arg Ile Tyr Lys Val Asp Gly Asn Ile Pro Phe Ala Leu Asn
   275                 280                 285                 290
   Thr Ala Asp Val Ser Leu Ser Thr Asp Gly Lys Thr Ile Thr Val Asp
                   295                 300                 305
   Ala Ser Thr Pro Phe Glu Asn Asn Thr Glu Tyr Lys Val Val Val Lys
               310                 315                 320
   Gly Ile Lys Asp Lys Asn Gly Lys Glu Phe Lys Glu Asp Ala Phe Thr
           325                 330                 335
   Phe Lys Leu Arg Asn Asp Ala Val Val Thr Gln Val Phe Gly Thr Asn
       340                 345                 350
   Val Thr Asn Asn Thr Ser Val Asn Leu Ala Ala Gly Thr Phe Asp Thr
   355                 360                 365                 370
   Asp Asp Thr Leu Thr Val Val Phe Asp Lys Leu Leu Ala Pro Glu Thr
                   375                 380                 385
   Val Asn Ser Ser Asn Val Thr Ile Thr Asp Val Glu Thr Gly Lys Arg
               390                 395                 400
   Ile Pro Val Ile Ala Ser Thr Ser Gly Ser Thr Ile Thr Ile Thr Leu
           405                 410                 415
   Lys Glu Ala Leu Val Thr Gly Lys Gln Tyr Lys Leu Ala Ile Asn Asn
       420                 425                 430
   Val Lys Thr Leu Thr Gly Tyr Asn Ala Glu Ala Tyr Glu Leu Val Phe
   435                 440                 445                 450
   Thr Ala Asn Ala Ser Ala Pro Thr Val Ala Thr Ala Pro Thr Thr Leu
                   455                 460                 465
   Gly Gly Thr Thr Leu Ser Thr Gly Ser Leu Thr Thr Asn Val Trp Gly
               470                 475                 480
   Lys Leu Ala Gly Gly Val Asn Glu Ala Gly Thr Tyr Tyr Pro Gly Leu
           485                 490                 495
   Gln Phe Thr Thr Thr Phe Ala Thr Lys Leu Asp Glu Ser Thr Leu Ala
       500                 505                 510
   Asp Asn Phe Val Leu Val Glu Lys Glu Ser Gly Thr Val Val Ala Ser
   515                 520                 525                 530
   Glu Leu Lys Tyr Asn Ala Asp Ala Lys Met Val Thr Leu Val Pro Lys
                   535                 540                 545
   Ala Asp Leu Lys Glu Asn Thr Ile Tyr Gln Ile Lys Ile Lys Lys Gly
               550                 555                 560
   Leu Lys Ser Asp Lys Gly Ile Glu Leu Gly Thr Val Asn Glu Lys Thr
           565                 570                 575
   Tyr Glu Phe Lys Thr Gln Asp Leu Thr Ala Pro Thr Val Ile Ser Val
       580                 585                 590
   Thr Ser Lys Asn Gly Asp Ala Gly Leu Lys Val Thr Glu Ala Gln Glu
   595                 600                 605                 610
   Phe Thr Val Lys Phe Ser Glu Asn Leu Asn Thr Phe Asn Ala Thr Thr
                   615                 620                 625
   Val Ser Gly Ser Thr Ile Thr Tyr Gly Gln Val Ala Val Val Lys Ala
               630                 635                 640
   Gly Ala Asn Leu Ser Ala Leu Thr Ala Ser Asp Ile Ile Pro Ala Ser
           645                 650                 655
   Val Glu Ala Val Thr Gly Gln Asp Gly Thr Tyr Lys Val Lys Val Ala
       660                 665                 670
   Ala Asn Gln Leu Glu Arg Asn Gln Gly Tyr Lys Leu Val Val Phe Gly
   675                 680                 685                 690
   Lys Gly Ala Thr Ala Pro Val Lys Asp Ala Ala Asn Ala Asn Thr Leu
                   695                 700                 705
   Ala Thr Asn Tyr Ile Tyr Thr Phe Thr Thr Glu Gly Gln Asp Val Thr
               710                 715                 720
   Ala Pro Thr Val Thr Lys Val Phe Lys Gly Asp Ser Leu Lys Asp Ala
   725 730 735
   Asp Ala Val Thr Thr Leu Thr Asn Val Asp Ala Gly Gln Lys Phe Thr
   740 745 750
   Ile Gln Phe Ser Glu Glu Leu Lys Thr Ser Ser Gly Ser Leu Val Gly
   755 760 765 770
   Gly Lys Val Thr Val Glu Lys Leu Thr Asn Asn Gly Trp Val Asp Ala
   775 780 785
   Gly Thr Gly Thr Thr Val Ser Val Ala Pro Lys Thr Asp Ala Asn Gly
   790 795 800
   Lys Val Thr Ala Ala Val Val Thr Leu Thr Gly Leu Asp Asn Asn Asp
   805 810 815
   Lys Asp Ala Lys Leu Arg Leu Val Val Asp Lys Ser Ser Thr Asp Gly
   820 825 830
   Ile Ala Asp Val Ala Gly Asn Val Ile Lys Glu Lys Asp Ile Leu Ile
   835 840 845 850
   Arg Tyr Asn Ser Trp Arg His Thr Val Ala Ser Val Lys Ala Ala Ala
   855 860 865
   Asp Lys Asp Gly Gln Asn Ala Ser Ala Ala Phe Pro Thr Ser Thr Ala
   870 875 880
   Ile Asp Thr Thr Lys Ser Leu Leu Val Glu Phe Asn Glu Thr Asp Leu
   885 890 895
   Ala Glu Val Lys Pro Glu Asn Ile Val Val Lys Asp Ala Ala Gly Asn
   900 905 910
   Ala Val Ala Gly Thr Val Thr Ala Leu Asp Gly Ser Thr Asn Lys Phe
   915 920 925 930
   Val Phe Thr Pro Ser Gln Glu Leu Lys Ala Gly Thr Val Tyr Ser Val
   935 940 945
   Thr Ile Asp Gly Val Arg Asp Lys Val Gly Asn Thr Ile Ser Lys Tyr
   950 955 960
   Ile Thr Ser Phe Lys Thr Val Ser Ala Asn Pro Thr Leu Ser Ser Ile
   965 970 975
   Ser Ile Ala Asp Gly Ala Val Asn Val Asp Arg Ser Lys Thr Ile Thr
   980 985 990
   Ile Glu Phe Ser Asp Ser Val Pro Asn Pro Thr Ile Thr Leu Lys Lys
   995 1000 1005 1010
   Ala Asp Gly Thr Ser Phe Thr Asn Tyr Thr Leu Val Asn Val Asn Asn
   1015 1020 1025
   Glu Asn Lys Thr Tyr Lys Ile Val Phe His Lys Gly Val Thr Leu Asp
   1030 1035 1040
   Glu Phe Thr Gln Tyr Glu Leu Ala Val Ser Lys Asp Phe Gln Thr Gly
   1045 1050 1055
   Thr Asp Ile Asp Ser Lys Val Thr Phe Ile Thr Gly Ser Val Ala Thr
   1060 1065 1070
   Asp Glu Val Lys Pro Ala Leu Val Gly Val Gly Ser Trp Asn Gly Thr
   1075                1080                1085                1090
   Ser Tyr Thr Gln Asp Ala Ala Ala Thr Arg Leu Arg Ser Val Ala Asp
                   1095                1100                1105
   Phe Val Ala Glu Pro Val Ala Leu Gln Phe Ser Glu Gly Ile Asp Leu
               1110                1115                1120
   Thr Asn Ala Thr Val Thr Val Thr Asn Ile Thr Asp Asp Lys Thr Val
           1125                1130                1135
   Glu Val Ile Ser Lys Glu Ser Val Asp Ala Asp His Asp Ala Gly Ala
       1140                1145                1150
   Thr Lys Glu Thr Leu Val Ile Asn Thr Val Thr Pro Leu Val Leu Asp
   1155                1160                1165                1170
   Asn Ser Lys Thr Tyr Lys Ile Val Val Ser Gly Val Lys Asp Ala Ala
                   1175                1180                1185
   Gly Asn Val Ala Asp Thr Ile Thr Phe Tyr Ile Lys
               1190                1195
(2) ANGABEN ZU SEQ ID NO: 3:
   (i) SEQUENZKENNZEICHEN:
      (A) LÄNGE: 2766 Basenpaare
      (B) ART: Nucleotid
      (C) STRANGFORM: beides
      (D) TOPOLOGIE: linear
   (vi) URSPRÜNGLICHE HERKUNFT:
      (A) ORGANISMUS: Bacillus stearothermophilus
      (B) STAMM: PV72
   (vii) UNMITTELBARE HERKUNFT:
      (B) CLON(E): sbsB
   (ix) MERKMAL:
      (A) NAME/SCHLÜSSEL: CDS
      (B) LAGE:1..2763
   (ix) MERKMAL:
      (A) NAME/SCHLÜSSEL: sig_peptide
      (B) LAGE:1..93
   (ix) MERKMAL:
      (A) NAME/SCHLÜSSEL: mat_eptide
      (B) LAGE:94..2763
   (xi) SEQUENZBESCHREIBUNG: SEQ ID NO: 3:
   ATG GCT TAT CAA CCT AAG TCT TTT CGC AAG TTT GTT GCG ACA ACT GCA          48
   Met Ala Tyr Gln Pro Lys Ser Phe Arg Lys Phe Val Ala Thr Thr Ala
   -31 -30                 -25                 -20
   ACA GCT GCC ATT GTA GCA TCT GCG GTA GCT CCT GTA GTA TCT GCA GCA          96
   Thr Ala Ala Ile Val Ala Ser Ala Val Ala Pro Val Val Ser Ala Ala
   -15                 -10                 -5                    1
   AGC TTC ACA GAT GTT GCG CCG CAA TAT AAA GAT GCG ATC GAT TTC TTA         144
   Ser Phe Thr Asp Val Ala Pro Gln Tyr Lys Asp Ala Ile Asp Phe Leu
                 5                  10                  15
   GTA TCA ACT GGT GCA ACA AAA GGT AAA ACA GAA ACA AAA TTC GGC GTT         192
   Val Ser Thr Gly Ala Thr Lys Gly Lys Thr Glu Thr Lys Phe Gly Val
            20                  25                  30
   TAC GAT GAA ATC ACT CGT CTA GAT GCG GCA GTT ATT CTT GCA AGA GTA         240
   Tyr Asp Glu Ile Thr Arg Leu Asp Ala Ala Val Ile Leu Ala Arg Val
        35                  40                  45
   TTA AAA CTA GAC GTT GAC AAC GCA AAA GAC GCA GGC TTC ACA GAT GTG         288
   Leu Lys Leu Asp Val Asp Asn Ala Lys Asp Ala Gly Phe Thr Asp Val
    50                  55                  60                  65
   CCA AAA GAC CGT GCA AAA TAC GTC AAC GCG CTT GTA GAA GCT GGC GTA         336
   Pro Lys Asp Arg Ala Lys Tyr Val Asn Ala Leu Val Glu Ala Gly Val
                    70                  75                  80
   TTA AAC GGT AAA GCA CCT GGC AAA TTT GGT GCA TAC GAC CCA TTA ACT         384
   Leu Asn Gly Lys Ala Pro Gly Lys Phe Gly Ala Tyr Asp Pro Leu Thr
                85                  90                  95
   CGC GTT GAA ATG GCA AAA ATC ATC GCG AAC CGT TAC AAA TTA AAA GCT         432
   Arg Val Glu Met Ala Lys Ile Ile Ala Asn Arg Tyr Lys Leu Lys Ala
           100                 105                 110
   GAC GAT GTA AAA CTT CCA TTC ACT GAT GTA AAC GAT ACA TGG GCA CCA         480
   Asp Asp Val Lys Leu Pro Phe Thr Asp Val Asn Asp Thr Trp Ala Pro
       115                 120                 125
   TAC GTA AAA GCG CTT TAT AAA TAC GAA GTA ACC AAA AGG TTA AAA CAC         528
   Tyr Val Lys Ala Leu Tyr Lys Tyr Glu Val Thr Lys Arg Leu Lys His
   130                 135                 140                 145
   CAA CAA GCT TCG GTG CAT ACC AAA AAC ATC ACT CTG CGT GAC TTT GCG         576
   Gln Gln Ala Ser Val His Thr Lys Asn Ile Thr Leu Arg Asp Phe Ala
                   150                 155                 160
   CAA TTT GTA TAT AGA GCG GTG AAT ATT AAT GCA GTG CCA GAA ATA GTT         624
   Gln Phe Val Tyr Arg Ala Val Asn Ile Asn Ala Val Pro Glu Ile Val
               165                 170                 175
   GAA GTA ACT GCG GTT AAT TCG ACT ACA GTG AAA GTA ACA TTC AAT ACG         672
   Glu Val Thr Ala Val Asn Ser Thr Thr Val Lys Val Thr Phe Asn Thr
           180                 185                 190
   CAA ATT GCT GAT GTT GAT TTC ACA AAT TTT GCT ATC GAT AAC GGT TTA         720
   Gln Ile Ala Asp Val Asp Phe Thr Asn Phe Ala Ile Asp Asn Gly Leu
       195                 200                 205
   ACT GTT ACT AAA GCA ACT CTT TCT CGT GAT AAA AAA TCC GTA GAG GTT         768
   Thr Val Thr Lys Ala Thr Leu Ser Arg Asp Lys Lys Ser Val Glu Val
   210                 215                 220                 225
   GTG GTA AAT AAA CCG TTT ACT CGT AAT CAG GAA TAT ACA ATT ACA GCG         816
   Val Val Asn Lys Pro Phe Thr Arg Asn Gln Glu Tyr Thr Ile Thr Ala
                   230                 235                 240
   ACA GGC ATT AAA AAT TTA AAA GGC GAG ACC GCT AAG GAA TTA ACT GGT        864
   Thr Gly Ile Lys Asn Leu Lys Gly Glu Thr Ala Lys Glu Leu Thr Gly
               245                 250                 255
   AAG TTT GTT TGG TCT GTT CAA GAT GCG GTA ACT GTT GCA CTA AAT AAT        912
   Lys Phe Val Trp Ser Val Gln Asp Ala Val Thr Val Ala Leu Asn Asn
           260                 265                 270
   AGT TCG CTT AAA GTT GGA GAG GAA TCT GGT TTA ACT GTA AAA GAT CAG        960
   Ser Ser Leu Lys Val Gly Glu Glu Ser Gly Leu Thr Val Lys Asp Gln
       275                 280                 285
   GAT GGC AAA GAT GTT GTA GGT GCT AAA GTA GAA CTT ACT TCT TCT AAT       1008
   Asp Gly Lys Asp Val Val Gly Ala Lys Val Glu Leu Thr Ser Ser Asn
   290                 295                 300                 305
   ACT AAT ATT GTT GTA GTT TCA AGT GGC GAA GTA TCA GTA TCT GCT GCT       1056
   Thr Asn Ile Val Val Val Ser Ser Gly Glu Val Ser Val Ser Ala Ala
                   310                 315                 320
   AAA GTT ACA GCT GTA AAA CCG GGA ACA GCT GAT GTT ACT GCA AAA GTT       1104
   Lys Val Thr Ala Val Lys Pro Gly Thr Ala Asp Val Thr Ala Lys Val
               325                 330                 335
   ACA TTA CCA GAT GGT GTT GTA CTA ACA AAT ACA TTT AAA GTG ACA GTT       1152
   Thr Leu Pro Asp Gly Val Val Leu Thr Asn Thr Phe Lys Val Thr Val
           340                 345                 350
   ACA GAA GTG CCT GTT CAA GTC CAA AAT CAA GGA TTT ACT TTA GTT GAT       1200
   Thr Glu Val Pro Val Gln Val Gln Asn Gln Gly Phe Thr Leu Val Asp
       355                 360                 365
   AAT CTT TCT AAT GCT CCA CAG AAT ACA GTT GCA TTT AAC AAA GCT GAG       1248
   Asn Leu Ser Asn Ala Pro Gln Asn Thr Val Ala Phe Asn Lys Ala Glu
   370                 375                 380                 385
   AAA GTA ACT TCA ATG TTT GCT GGA GAA ACT AAA ACA GTT GCA ATG TAT       1296
   Lys Val Thr Ser Met Phe Ala Gly Glu Thr Lys Thr Val Ala Met Tyr
                   390                 395                 400
   GAT ACT AAA AAC GGT GAT CCT GAA ACT AAA CCT GTT GAT TTC AAA GAT       1344
   Asp Thr Lys Asn Gly Asp Pro Glu Thr Lys Pro Val Asp Phe Lys Asp
               405                 410                 415
   GCA ACT GTA CGT TCA TTA AAT CCA ATT ATT GCA ACA GCT GCT ATT AAT       1392
   Ala Thr Val Arg Ser Leu Asn Pro Ile Ile Ala Thr Ala Ala Ile Asn
           420                 425                 430
   GGT AGT GAG CTC CTT GTC ACA GCT AAT GCT GGC CAA TCT GGA AAA GCT       1440
   Gly Ser Glu Leu Leu Val Thr Ala Asn Ala Gly Gln Ser Gly Lys Ala
       435                 440                 445
   TCA TTT GAA GTA ACA TTA AAA GAT AAT ACA AAA AGA ACA TTT ACA GTT       1488
   Ser Phe Glu Val Thr Leu Lys Asp Asn Thr Lys Arg Thr Phe Thr Val
   450                 455                 460                 465
   GAT GTA AAA AAA GAC CCT GTA TTA CAA GAT ATA AAA GTA GAT GCA ACT       1536
   Asp Val Lys Lys Asp Pro Val Leu Gln Asp Ile Lys Val Asp Ala Thr
                   470                 475                 480
   TCT GTT AAA CTT TCC GAT GAA GCT GTT GGC GGC GGG GAA GTT GAA GGA       1584
   Ser Val Lys Leu Ser Asp Glu Ala Val Gly Gly Gly Glu Val Glu Gly
               485                 490                 495
   GTT AAC CAA AAA ACG ATT AAA GTA AGT GCA GTT GAC CAA TAC GGT AAA       1632
   Val Asn Gln Lys Thr Ile Lys Val Ser Ala Val Asp Gln Tyr Gly Lys
           500                 505                 510
   GAA ATT AAA TTT GGT ACA AAA GGT AAA GTT ACT GTT ACA ACT AAT ACA      1680
   Glu Ile Lys Phe Gly Thr Lys Gly Lys Val Thr Val Thr Thr Asn Thr
       515                 520                 525
   GAA GGA CTA GTT ATT AAA AAT GTA AAT AGC GAT AAT ACA ATT GAC TTT      1728
   Glu Gly Leu Val Ile Lys Asn Val Asn Ser Asp Asn Thr Ile Asp Phe
   530                 535                 540                 545
   GAT AGC GGC AAT AGT GCA ACT GAC CAA TTT GTT GTC GTT GCA ACA AAA      1776
   Asp Ser Gly Asn Ser Ala Thr Asp Gln Phe Val Val Val Ala Thr Lys
                   550                 555                 560
   GAC AAA ATT GTC AAT GGT AAA GTA GAA GTT AAA TAT TTC AAA AAT GCT      1824
   Asp Lys Ile Val Asn Gly Lys Val Glu Val Lys Tyr Phe Lys Asn Ala
               565                 570                 575
   AGT GAC ACA ACA CCA ACT TCA ACT AAA ACA ATT ACT GTT AAT GTA GTA      1872
   Ser Asp Thr Thr Pro Thr Ser Thr Lys Thr Ile Thr Val Asn Val Val
           580                 585                 590
   AAT GTA AAA GCT GAC GCT ACA CCA GTA GGA TTA GAT ATT GTA GCA CCT      1920
   Asn Val Lys Ala Asp Ala Thr Pro Val Gly Leu Asp Ile Val Ala Pro
       595                 600                 605
   TCT AAA ATT GAT GTA AAT GCT CCA AAC ACT GCT TCT ACT GCA GAT GTT      1968
   Ser Lys Ile Asp Val Asn Ala Pro Asn Thr Ala Ser Thr Ala Asp Val
   610                 615                 620                 625
   GAT TTT ATA AAT TTC GAA AGT GTT GAG ATT TAC ACA CTC GAT TCA AAT      2016
   Asp Phe Ile Asn Phe Glu Ser Val Glu Ile Tyr Thr Leu Asp Ser Asn
                   630                 635                 640
   GGT AGA CGT CAA AAA AAA GTT ACT CCA ACT GCA ACT ACA CTT GTA GGT      2064
   Gly Arg Arg Gln Lys Lys Val Thr Pro Thr Ala Thr Thr Leu Val Gly
               645                 650                 655
   ACA AAA AAA AAA AAA AAA GTT AAT GGG AAT GTA TTA CAA TTC AAG GGG      2112
   Thr Lys Lys Lys Lys Lys Val Asn Gly Asn Val Leu Gln Phe Lys Gly
           660                 665                 670
   AAC GAA GAA TTA ACG CTA TCA ACT TCT TCT AGT ACA GGA AAC GTA GAT      2160
   Asn Glu Glu Leu Thr Leu Ser Thr Ser Ser Ser Thr Gly Asn Val Asp
       675                 680                 685
   GGA ACA GCA GAA GGA ATG ACA AAA CGT ATT CCA GGG AAA TAT ATC AAC      2208
   Gly Thr Ala Glu Gly Met Thr Lys Arg Ile Pro Gly Lys Tyr Ile Asn
   690                 695                 700                 705
   TCT GCA AGT GTA CCT GCC AGT GCA ACA GTA GCA ACA AGT CCT GTT ACT      2256
   Ser Ala Ser Val Pro Ala Ser Ala Thr Val Ala Thr Ser Pro Val Thr
                   710                 715                 720
   GTA AAG CTT AAT TCA AGT GAT AAT GAT TTA ACA TTT GAA GAA TTA ATA      2304
   Val Lys Leu Asn Ser Ser Asp Asn Asp Leu Thr Phe Glu Glu Leu Ile
               725                 730                 735
   TTC GGT GTA ATT GAC CCT ACA CAA TTA GTC AAA GAT GAA GAC ATC AAC      2352
   Phe Gly Val Ile Asp Pro Thr Gln Leu Val Lys Asp Glu Asp Ile Asn
           740                 745                 750
   GAA TTT ATT GCA GTT TCA AAA GCG GCT AAA AAT GAT GGA TAT TTG TAT      2400
   Glu Phe Ile Ala Val Ser Lys Ala Ala Lys Asn Asp Gly Tyr Leu Tyr
       755                 760                 765
   AAT AAA CCG CTT GTA ACG GTT AAA GAT GCA TCA GGA AAA GTT ATT CCA      2448
   Asn Lys Pro Leu Val Thr Val Lys Asp Ala Ser Gly Lys Val Ile Pro
   770                 775                 780                 785
   ACA GGT GCA AAT GTT TAC GGT CTA AAT CAT GAT GCA ACT AAC GGA AAC      2496
   Thr Gly Ala Asn Val Tyr Gly Leu Asn His Asp Ala Thr Asn Gly Asn
                   790                 795                 800
   ATT TGG TTT GAT GAG GAA CAA GCT GGC TTA GCT AAA AAA TTT AGT GAT      2544
   Ile Trp Phe Asp Glu Glu Gln Ala Gly Leu Ala Lys Lys Phe Ser Asp
               805                 810                 815
   GTA CAT TTT GAT GTT GAT TTT TCA TTA ACT AAC GTT GTA AAA ACT GGT      2592
   Val His Phe Asp Val Asp Phe Ser Leu Thr Asn Val Val Lys Thr Gly
           820                 825                 830
   AGC GGT ACA GTT TCT TCA TCG CCA TCA TTA TCT GAC GCA ATT CAA CTT      2640
   Ser Gly Thr Val Ser Ser Ser Pro Ser Leu Ser Asp Ala Ile Gln Leu
       835                 840                 845
   ACT AAT TCA GGC GAT GCA GTA TCG TTT ACA TTA GTT ATC AAA TCA ATT      2688
   Thr Asn Ser Gly Asp Ala Val Ser Phe Thr Leu Val Ile Lys Ser Ile
   850                 855                 860                 865
   TAT GTT AAA GGC GCA GAT AAA GAT GAT AAT AAC TTA CTT GCA GCC CCT      2736
   Tyr Val Lys Gly Ala Asp Lys Asp Asp Asn Asn Leu Leu Ala Ala Pro
                   870                 875                 880
   GTT TCT GTC AAT GTG ACT GTG ACA AAA TAA                              2766
   Val Ser Val Asn Val Thr Val Thr Lys
               885                 890
(2) ANGABEN ZU SEQ ID NO: 4:
   (i) SEQUENZKENNZEICHEN:
      (A) LÄNGE: 921 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 4:
   Met Ala Tyr Gln Pro Lys Ser Phe Arg Lys Phe Val Ala Thr Thr Ala
   -31 -30                 -25                 -20
   Thr Ala Ala Ile Val Ala Ser Ala Val Ala Pro Val Val Ser Ala Ala
   -15                 -10                  -5                   1
   Ser Phe Thr Asp Val Ala Pro Gln Tyr Lys Asp Ala Ile Asp Phe Leu
                     5                  10                  15
   Val Ser Thr Gly Ala Thr Lys Gly Lys Thr Glu Thr Lys Phe Gly Val
               20                  25                  30
   Tyr Asp Glu Ile Thr Arg Leu Asp Ala Ala Val Ile Leu Ala Arg Val
           35                  40                  45
   Leu Lys Leu Asp Val Asp Asn Ala Lys Asp Ala Gly Phe Thr Asp Val
    50                  55                  60                  65
   Pro Lys Asp Arg Ala Lys Tyr Val Asn Ala Leu Val Glu Ala Gly Val
                    70                  75                  80
   Leu Asn Gly Lys Ala Pro Gly Lys Phe Gly Ala Tyr Asp Pro Leu Thr
                85                  90                  95
   Arg Val Glu Met Ala Lys Ile Ile Ala Asn Arg Tyr Lys Leu Lys Ala
           100                 105                 110
   Asp Asp Val Lys Leu Pro Phe Thr Asp Val Asn Asp Thr Trp Ala Pro
       115                 120                 125
   Tyr Val Lys Ala Leu Tyr Lys Tyr Glu Val Thr Lys Arg Leu Lys His
   130                 135                 140                 145
   Gln Gln Ala Ser Val His Thr Lys Asn Ile Thr Leu Arg Asp Phe Ala
                   150                 155                 160
   Gln Phe Val Tyr Arg Ala Val Asn Ile Asn Ala Val Pro Glu Ile Val
               165                 170                 175
   Glu Val Thr Ala Val Asn Ser Thr Thr Val Lys Val Thr Phe Asn Thr
           180                 185                 190
   Gln Ile Ala Asp Val Asp Phe Thr Asn Phe Ala Ile Asp Asn Gly Leu
       195                 200                 205
   Thr Val Thr Lys Ala Thr Leu Ser Arg Asp Lys Lys Ser Val Glu Val
   210                 215                 220                 225
   Val Val Asn Lys Pro Phe Thr Arg Asn Gln Glu Tyr Thr Ile Thr Ala
                   230                 235                 240
   Thr Gly Ile Lys Asn Leu Lys Gly Glu Thr Ala Lys Glu Leu Thr Gly
               245                 250                 255
   Lys Phe Val Trp Ser Val Gln Asp Ala Val Thr Val Ala Leu Asn Asn
           260                 265                 270
   Ser Ser Leu Lys Val Gly Glu Glu Ser Gly Leu Thr Val Lys Asp Gln
       275                 280                 285
   Asp Gly Lys Asp Val Val Gly Ala Lys Val Glu Leu Thr Ser Ser Asn
   290                 295                 300                 305
   Thr Asn Ile Val Val Val Ser Ser Gly Glu Val Ser Val Ser Ala Ala
                   310                 315                 320
   Lys Val Thr Ala Val Lys Pro Gly Thr Ala Asp Val Thr Ala Lys Val
               325                 330                 335
   Thr Leu Pro Asp Gly Val Val Leu Thr Asn Thr Phe Lys Val Thr Val
           340                 345                 350
   Thr Glu Val Pro Val Gln Val Gln Asn Gln Gly Phe Thr Leu Val Asp
       355                 360                 365
   Asn Leu Ser Asn Ala Pro Gln Asn Thr Val Ala Phe Asn Lys Ala Glu
   370                 375                 380                 385
   Lys Val Thr Ser Met Phe Ala Gly Glu Thr Lys Thr Val Ala Met Tyr
                   390                 395                 400
   Asp Thr Lys Asn Gly Asp Pro Glu Thr Lys Pro Val Asp Phe Lys Asp
               405                 410                 415
   Ala Thr Val Arg Ser Leu Asn Pro Ile Ile Ala Thr Ala Ala Ile Asn
           420                 425                 430
   Gly Ser Glu Leu Leu Val Thr Ala Asn Ala Gly Gln Ser Gly Lys Ala
       435                 440                 445
   Ser Phe Glu Val Thr Leu Lys Asp Asn Thr Lys Arg Thr Phe Thr Val
   450                 455                 460                 465
   Asp Val Lys Lys Asp Pro Val Leu Gln Asp Ile Lys Val Asp Ala Thr
                   470                 475                 480
   Ser Val Lys Leu Ser Asp Glu Ala Val Gly Gly Gly Glu Val Glu Gly
               485                 490                 495
   Val Asn Gln Lys Thr Ile Lys Val Ser Ala Val Asp Gln Tyr Gly Lys
           500                 505                 510
   Glu Ile Lys Phe Gly Thr Lys Gly Lys Val Thr Val Thr Thr Asn Thr
       515                 520                 525
   Glu Gly Leu Val Ile Lys Asn Val Asn Ser Asp Asn Thr Ile Asp Phe
   530                 535                 540                 545
   Asp Ser Gly Asn Ser Ala Thr Asp Gln Phe Val Val Val Ala Thr Lys
                   550                 555                 560
   Asp Lys Ile Val Asn Gly Lys Val Glu Val Lys Tyr Phe Lys Asn Ala
               565                 570                 575
   Ser Asp Thr Thr Pro Thr Ser Thr Lys Thr Ile Thr Val Asn Val Val
           580                 585                 590
   Asn Val Lys Ala Asp Ala Thr Pro Val Gly Leu Asp Ile Val Ala Pro
       595                 600                 605
   Ser Lys Ile Asp Val Asn Ala Pro Asn Thr Ala Ser Thr Ala Asp Val
   610                 615                 620                 625
   Asp Phe Ile Asn Phe Glu Ser Val Glu Ile Tyr Thr Leu Asp Ser Asn
                   630                 635                 640
   Gly Arg Arg Gln Lys Lys Val Thr Pro Thr Ala Thr Thr Leu Val Gly
               645                 650                 655
   Thr Lys Lys Lys Lys Lys Val Asn Gly Asn Val Leu Gln Phe Lys Gly
           660                 665                 670
   Asn Glu Glu Leu Thr Leu Ser Thr Ser Ser Ser Thr Gly Asn Val Asp
       675                 680                 685
   Gly Thr Ala Glu Gly Met Thr Lys Arg Ile Pro Gly Lys Tyr Ile Asn
   690                 695                 700                 705
   Ser Ala Ser Val Pro Ala Ser Ala Thr Val Ala Thr Ser Pro Val Thr
                   710                 715                 720
   Val Lys Leu Asn Ser Ser Asp Asn Asp Leu Thr Phe Glu Glu Leu Ile
               725                 730                 735
   Phe Gly Val Ile Asp Pro Thr Gln Leu Val Lys Asp Glu Asp Ile Asn
           740                 745                 750
   Glu Phe Ile Ala Val Ser Lys Ala Ala Lys Asn Asp Gly Tyr Leu Tyr
       755                 760                 765
   Asn Lys Pro Leu Val Thr Val Lys Asp Ala Ser Gly Lys Val Ile Pro
   770                 775                 780                 785
   Thr Gly Ala Asn Val Tyr Gly Leu Asn His Asp Ala Thr Asn Gly Asn
                   790                 795                 800
   Ile Trp Phe Asp Glu Glu Gln Ala Gly Leu Ala Lys Lys Phe Ser Asp
   805 810 815
   Val His Phe Asp Val Asp Phe Ser Leu Thr Asn Val Val Lys Thr Gly
   820 825 830
   Ser Gly Thr Val Ser Ser Ser Pro Ser Leu Ser Asp Ala Ile Gln Leu
   835 840 845
   Thr Asn Ser Gly Asp Ala Val Ser Phe Thr Leu Val Ile Lys Ser Ile
   850 855 860 865
   Tyr Val Lys Gly Ala Asp Lys Asp Asp Asn Asn Leu Leu Ala Ala Pro
   870 875 880
   Val Ser Val Asn Val Thr Val Thr Lys
   885 890